# EUROPEAN PATENT APPLICATION

(11) **EP 2 859 831 A1**
(43) Date of publication of application: **15.04.2015**
(21) Application number: 13800334.8
(22) Date of filing: 10.06.2013
(51) Int. Cl.: A61B 1/00

(54) **CAPSULE-TYPE ENDOSCOPE DEVICE, RECEPTION DEVICE, AND CAPSULE-TYPE ENDOSCOPE SYSTEM**

(30) Priority: 08.06.2012 JP 2012131195
(71) Applicant: Olympus Medical Systems Corp., Tokyo 151-0072 (JP)
(72) Inventor: KIMOTO, Seiichiro, Tokyo 151-0072 (JP); KOIDE, Naoto, Tokyo 151-0072 (JP); MIYAZONO, Toru, Tokyo 151-0072 (JP)
(74) Representative: Gunzelmann, Rainer
(86) International application number: PCT/JP2013/065948
(87) International publication number: WO 2013/183785

(57) **Abstract**

Provided is a capsule endoscope apparatus, receiving apparatus, and capsule endoscope system that can reduce the consumption of a battery. A capsule endoscope apparatus 3 to be introduced into a subject includes an imaging unit 303 that captures an image of the subject, and generates image data of the inside of the subject, a transmission unit 306 that transmits a wireless signal including the image data to the outside, a receiving unit 308 that receives, from the outside, a control signal to instruct the operation of the capsule endoscope apparatus 3, and a capsule controller 312 that activates the receiving unit 308 in synchronization with a transmission timing when the transmission unit 306 transmits the wireless signal.

## Description

### Field

The present invention relates to a capsule endoscope apparatus configured to be introduced into a subject to capture an image of an inside of the subject, a receiving apparatus for receiving information transmitted from the capsule endoscope apparatus, and a capsule endoscope system.

### Background

In recent years, a capsule endoscope apparatus has been known which includes, in a capsule-shaped casing, an imaging device, a communication device that wirelessly transmits image data taken by the imaging device to the outside, and the like.

The capsule endoscope apparatus is swallowed from the mouth of a subject to observe the body cavity of the subject, and then moves through the inside of the organs such as the esophagus, stomach, and small intestine in accordance with the peristaltic movement of the organs to sequentially capture images until being naturally excreted from the subject. During the movement in the body cavity, the image data captured in the body cavity by the capsule endoscope is sequentially transmitted to the outside of the body in wireless communication, and stored in a memory provided inside or outside a receiving apparatus located outside the body.

A doctor or nurse transfers image data stored in the memory to an image processing apparatus via a cradle in which the receiving apparatus has been inserted, and diagnoses the subject based on an image displayed on a display monitor of the image processing apparatus.

If a wireless signal is received from the capsule endoscope apparatus, in a general receiving apparatus, a plurality of receiving antennas is distributed and placed outside the subject, one antenna having the strongest reception strength to receive is selected, and the selected antenna receives a wireless signal. For example, a technology is disclosed in which a receiving apparatus switches reception among a plurality of antennas placed outside a subject, and detects the position of a capsule endoscope apparatus inside the subject, the capsule endoscope apparatus being a transmission source of a wireless signal, based on the electric field strength received by each antenna (see Patent Literature 1).

### Citation List

### Patent Literature

Patent Literature 1: Japanese Laid-open Patent Publication No. 2007-608

### Summary

### Technical Problem

The above-mentioned capsule endoscope apparatus switches an operating mode that changes a shooting interval and an illumination method, according to a control signal transmitted from the receiving apparatus. However, the above-mentioned capsule endoscope apparatus always supplies power to a receiving unit that receives the control signal via an antenna in order to receive the control signal transmitted from the receiving apparatus, and is put in a state capable of receiving the control signal. Hence, there is a problem that a large amount of power of a battery mounted in the capsule endoscope apparatus is consumed.

The present invention has been made in view of the foregoing, and an object of the invention is to provide a capsule endoscope apparatus, receiving apparatus, and capsule endoscope system that can reduce the consumption of a battery.

### Solution to Problem

To solve the above-mentioned problem and achieve the object, a capsule endoscope apparatus according to the invention is a capsule endoscope apparatus that is configured to be introduced into a subject to acquire information on an inside of the subject and to wirelessly communicate with a receiving apparatus provided outside the capsule endoscope apparatus. The capsule endoscope apparatus includes: an imaging unit configured to capture images of the subject and generate image data of the inside of the subject; a transmission unit configured to transmit a wireless signal including the image data, outside the capsule endoscope apparatus; a receiving unit configured to receive a control signal for instructing an operation of the capsule endoscope apparatus, the control signal having been transmitted from the receiving apparatus; and a capsule controller configured to activate the receiving unit in synchronization with a transmission timing when the transmission unit transmits the wireless signal.

According to the capsule endoscope apparatus of the invention, in the above invention, the capsule controller activates the receiving unit immediately after the transmission unit starts transmitting the wireless signal.

According to the capsule endoscope apparatus of the invention, in the above invention, the capsule controller activates the receiving unit after the transmission unit completes transmission of the wireless signal.

According to the capsule endoscope apparatus of the invention, in the above invention, the imaging unit is configured to capture the images at a specified frame rate, and the capsule controller is configured to decide the number of times of activation to activate the receiving unit in accordance with the specified frame rate.

According to the capsule endoscope apparatus of the invention, in the above invention, the capsule controller is configured to cause the imaging unit to halt image capture at specified frame intervals, and activate the receiving unit in a pause period during which the imaging unit pauses.

According to the capsule endoscope apparatus of the invention, in the above invention, when the receiving unit receives the control signal having the same instruction content a plurality of times, the capsule controller causes the capsule endoscope apparatus to execute an operation in accordance with the control signal only once.

A capsule endoscope system according to the invention includes: a capsule endoscope apparatus configured to be introduced into a subject to acquire information on an inside of the subject; and a receiving apparatus configured to wirelessly communicate with the capsule endoscope apparatus. The capsule endoscope apparatus includes: an imaging unit configured to capture images of the subject and generate image data of the inside of the subject; a transmission unit configured to transmit a wireless signal including the image data, outside the capsule endoscope apparatus; a receiving unit configured to receive a control signal from the receiving apparatus; and a capsule controller configured to activate the receiving unit in synchronization with a transmission timing when the transmission unit transmits the wireless signal. The receiving apparatus includes: a receiving unit configured to receive the wireless signal; a transmission unit configured to transmit the control signal; and a reception controller configured to cause the transmission unit to transmit the control signal having the same instruction content a plurality of times at specified intervals immediately after the receiving unit receives the wireless signal.

A receiving apparatus according to the invention is a receiving apparatus to which an antenna apparatus having a plurality of antennas is detachably attached. The receiving apparatus includes: an antenna selector configured to select one antenna from among the plurality of antennas for receiving a wireless signal transmitted from outside when the antenna apparatus is attached; and a break determination unit configured to determine whether or not a break occurs in the antenna selected by the antenna selector.

In the above invention, the receiving apparatus according to the invention includes: a display unit configured to display an image; and a display controller configured to cause the display unit to display break information indicating that the break occurs in the selected antenna if the break determination unit determines that the break occurs in the selected antenna.

In the above invention, the receiving apparatus according to the invention further includes an operation input unit configured to receive input of an instruction signal to delete the break information. The display controller causes the display unit to delete only the break information when the instruction signal is input.

A receiving apparatus according to the invention is a receiving apparatus for receiving a wireless signal having information on an inside of a subject transmitted from a capsule endoscope apparatus introduced into the subject. The receiving apparatus includes: a power supply unit configured to supply power to component parts of the receiving apparatus; a display unit configured to display an image corresponding to image data; a remaining capacity detection unit configured to detect remaining capacity stored in the power supply unit; a remaining capacity determination unit configured to determine whether or not the remaining capacity detected by the remaining capacity detection unit is equal to or more than a preset threshold value; and a display controller configured to cause the display unit to display a warning when the remaining capacity determination unit determines that the remaining capacity is less than the threshold value.

In the above invention, the receiving apparatus according to the invention further includes a power measurement unit configured to measure power consumption consumed by the display unit. The remaining capacity determination unit is configured to determine whether or not remaining capacity obtained by subtracting the power consumption measured by the power measurement unit from the remaining capacity detected by the remaining capacity detection unit at a start of an examination is equal to or more than the threshold value.

According to the receiving apparatus of the invention, in the above invention, the power measurement unit starts measuring the power consumption when the remaining capacity determination unit determines that the remaining capacity is less than the threshold value.

In the above invention, the receiving apparatus according to the invention further includes an operating mode switching unit configured to switch to one of an arbitrary operating mode to allow an arbitrary operation and a restriction mode in which operation is restricted, in order to set for the receiving apparatus. The operating mode switching unit sets the receiving apparatus in the restriction mode when the remaining capacity determination unit determines that the remaining capacity of the power supply unit is less than the threshold value.

In the above invention, the receiving apparatus according to the invention further includes an operation input unit configured to receive input of an instruction signal to set a setting condition for the receiving apparatus before the start of the examination. The operating mode switching unit switches from the restriction mode to the arbitrary operating mode when the receiving apparatus satisfies the setting condition.

In the above invention, the receiving apparatus according to the invention further includes a recording unit configured to record the image data. The setting condition is any of data amount of the image data to be recorded by the recording unit, the remaining capacity of the power supply unit, and elapsed time from execution of the examination.

### Advantageous Effects of Invention

According to the invention, a capsule controller activates a receiving unit in synchronization with transmission timing when a transmission unit transmits a wireless signal. Accordingly, consumption of a battery can be reduced.

### Brief Description of Drawings

FIG. 1 is a schematic diagram illustrating a general configuration of a capsule endoscope system according to a first embodiment of the present invention.
FIG. 2 is a block diagram illustrating a general configuration of a capsule endoscope apparatus according to the first embodiment of the present invention.
FIG. 3 is a schematic diagram illustrating configurations of an antenna apparatus and an antenna cable according to the first embodiment of the present invention.
FIG. 4 is a block diagram illustrating functional configurations of the antenna apparatus and an antenna connection unit according to the first embodiment of the present invention.
FIG. 5 is a schematic diagram illustrating the appearance of a receiving apparatus according to the first embodiment of the present invention.
FIG. 6 is a block diagram illustrating a functional configuration of the receiving apparatus according to the first embodiment of the present invention.
FIG. 7 is a timing chart illustrating a relationship between transmission timings when the capsule endoscope apparatus according to the first embodiment of the present invention transmits image data and transmission timings when the receiving apparatus transmits a control signal.
FIG. 8 is a flowchart illustrating an outline of a process to be executed by the capsule endoscope apparatus according to the first embodiment of the present invention.
FIG. 9 is a diagram illustrating an example of a configuration of a wireless signal to be transmitted by the capsule endoscope apparatus according to the first embodiment of the present invention.
FIG. 10 is a block diagram illustrating a functional configuration of a capsule endoscope apparatus according to a second embodiment of the present invention.
FIG. 11 is a timing chart illustrating a relationship between transmission timings when the capsule endoscope apparatus according to the second embodiment of the present invention transmits image data and transmission timings when a receiving apparatus transmits a control signal.
FIG. 12 is a timing chart illustrating a relationship between transmission timings when a capsule endoscope apparatus according to a third embodiment of the present invention transmits image data and transmission timings when a receiving apparatus transmits a control signal.
FIG. 13 is a schematic diagram illustrating general configurations of a receiving apparatus, a cradle, and an image processing apparatus according to a fourth embodiment of the present invention.
FIG. 14 is a block diagram illustrating functional configurations of the receiving apparatus and the cradle according to the fourth embodiment of the present invention.
FIG. 15 is a diagram illustrating an example of an event information setting screen for guidance by the receiving apparatus, the event information setting screen being displayed on a display unit of the image processing apparatus according to the fourth embodiment of the present invention.
FIG. 16 is a diagram illustrating an example of an image displayed on a display unit of the receiving apparatus according to the fourth embodiment of the present invention.
FIG. 17 is a diagram illustrating an example of a time table of an initial setting process and occurrence of an event of the receiving apparatus according to the fourth embodiment of the present invention.
FIG. 18 is a diagram illustrating an example of a menu screen displayed on the display unit of the receiving apparatus according to the fourth embodiment of the present invention.
FIG. 19 is a diagram illustrating an example of a display screen displayed on the display unit of the receiving apparatus according to the fourth embodiment of the present invention.
FIG. 20 is a block diagram illustrating functional configurations of a receiving apparatus and a cradle according to a first modification of the fourth embodiment of the present invention.
FIG. 21 is a flowchart illustrating an outline of a process to be executed by a receiving apparatus according to a fifth embodiment of the present invention.
FIG. 22 is a diagram illustrating timings when the receiving apparatus according to the fifth embodiment of the present invention acquires information from an antenna connection unit.
FIG. 23 is a diagram illustrating an example of an error message displayed on a display unit of the receiving apparatus according to the fifth embodiment of the present invention.
FIG. 24 is a diagram illustrating an example of a display screen displayed on the display unit of the receiving apparatus according to the fifth embodiment of the present invention.
FIG. 25 is a flowchart illustrating an outline of a power determination process to be executed by a receiving apparatus according to a sixth embodiment of the present invention.
FIG. 26 is a schematic diagram illustrating an outline of power of a power supply unit of the receiving apparatus according to the sixth embodiment of the present invention.
FIG. 27 is a diagram illustrating an example of an attention message displayed on a display unit of the receiving apparatus according to the sixth embodiment of the present invention.
FIG. 28 is a diagram illustrating an example of a warning message displayed on the display unit of the receiving apparatus according to the sixth embodiment of the present invention.
FIG. 29 is a diagram illustrating an example of a forbidden message displayed on the display unit of the receiving apparatus according to the sixth embodiment of the present invention.
FIG. 30 is a diagram illustrating examples of screen transition in a setting method for setting a capture image while a display unit of a receiving apparatus according to a seventh embodiment of the present invention is displaying a real time view image.
FIG. 31 is a diagram illustrating examples of screen transition in the setting method for setting a capture image while the display unit of the receiving apparatus according to the seventh embodiment of the present invention is displaying a real time view image.
FIG. 32 is a diagram illustrating examples of screen transition in the setting method for setting a capture image while the display unit of the receiving apparatus according to the seventh embodiment of the present invention is displaying a real time view image.
FIG. 33 is a diagram illustrating an example of an examination screen of a subject displayed by an image processing apparatus according to the seventh embodiment of the present invention.
FIG. 34 is a diagram illustrating an example of a playback view screen displayed in playback view mode by the receiving apparatus according to the seventh embodiment of the present invention.
FIG. 35 is a diagram illustrating another example of the playback view screen displayed in playback view mode by the receiving apparatus according to the seventh embodiment of the present invention.
FIG. 36 is a diagram illustrating examples of screen transition of the playback view screen displayed in playback view mode by the receiving apparatus according to the seventh embodiment of the present invention.

### Description of Embodiments

A capsule endoscope apparatus, a receiving apparatus, and a capsule endoscope system according to embodiments of the present invention (hereinafter referred to as "embodiments") will be described below with reference to the drawings. The present invention is not limited by the embodiments. The same reference numerals will be used to refer to the same elements.

### (First Embodiment)

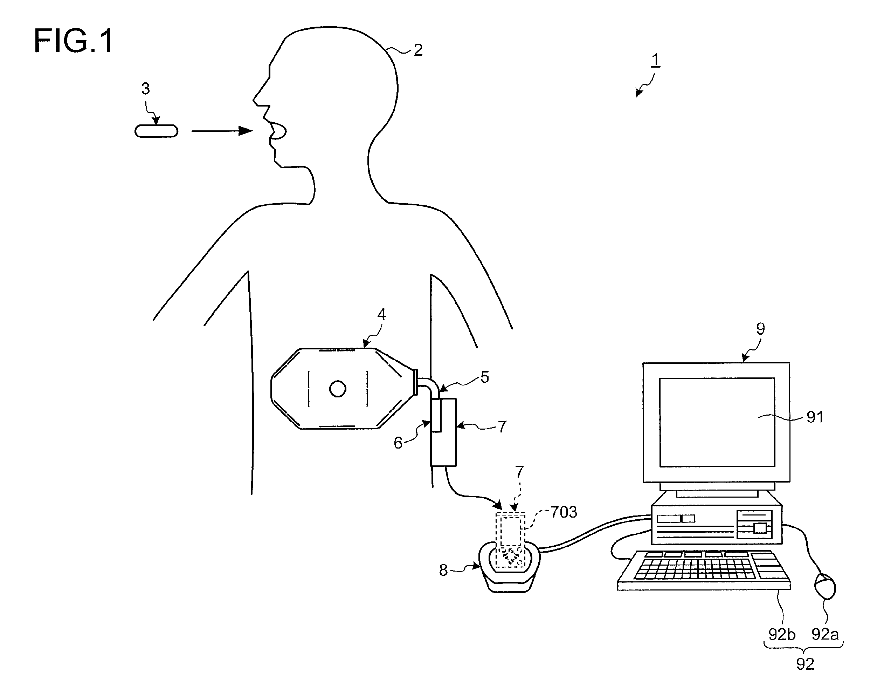

FIG. 1 is a schematic diagram illustrating a general configuration of a capsule endoscope system according to a first embodiment of the present invention.

As illustrated in FIG. 1, a capsule endoscope system 1 includes a capsule endoscope apparatus 3 that captures an in-vivo image inside a subject 2, an antenna apparatus 4 that receives a wireless signal transmitted from the capsule endoscope apparatus 3 introduced into the subject 2, an antenna connection unit 6 that performs specified processes on a wireless signal input from the antenna apparatus 4 via an antenna cable 5, a receiving apparatus 7 that performs specified processes on a signal input from the antenna connection unit 6, and records image data captured by the capsule endoscope apparatus 3 or displays an image corresponding to the image data, and an image processing apparatus 9 that is realized by a workstation, personal computer, or the like that includes a display screen such as a monitor, performs specified image processing on the image data of the inside of the subject 2 recorded in the receiving apparatus 7 via a cradle 8, and displays an image of the inside of the subject 2. The antenna apparatus 4 is inserted into an unillustrated antenna holder and attached to the subject 2. Moreover, the receiving apparatus 7 is inserted into an unillustrated receiving apparatus holder and attached to the subject 2.

The capsule endoscope apparatus 3 has an imaging function to capture an image inside the subject 2, and a wireless communication function to convert image data obtained by capturing an image inside the subject 2 into a wireless signal and transmit the wireless signal to the antenna apparatus 4, and receive a wireless signal transmitted from the antenna apparatus 4. Being swallowed into the subject 2, the capsule endoscope apparatus 3 passes through the esophagus in the subject 2 and moves in the body cavity by the peristaltic movement of digestive lumen. The capsule endoscope apparatus 3 consecutively captures images inside the body cavity of the subject 2 at minute time intervals, for example, at intervals of 0.5 seconds while moving in the body cavity, and generates the captured image data of the inside of the subject 2 to sequentially transmit the image data to the antenna apparatus 4. In this case, the capsule endoscope apparatus 3 generates a transmission signal including the image data, and reception strength detection data such as location information (beacon) that facilitates the detection of reception strength, and wirelessly transmits, to the antenna apparatus 4, a wireless signal that can be obtained by modulating the generated transmission signal.

The antenna apparatus 4 receives a wireless signal periodically from the capsule endoscope apparatus 3, and outputs the wireless signal to the antenna connection unit 6 via the antenna cable 5. Moreover, the antenna apparatus 4 transmits a control signal input from the antenna connection unit 6 via the antenna cable 5, to the capsule endoscope apparatus 3 in the subject 2.

The antenna cable 5 is configured using a coaxial cable. The antenna cable 5 propagates, to the antenna connection unit 6, a wireless signal received by the antenna apparatus 4. Moreover, the antenna cable 5 propagates, to the antenna apparatus 4, a control signal input from the antenna connection unit 6.

The antenna connection unit 6 is detachable from the receiving apparatus 7. The antenna connection unit 6 extracts the image data of the inside of the subject 2 based on the wireless signal transmitted from the capsule endoscope apparatus 3 via the antenna apparatus 4 and the antenna cable 5, and detects reception strength responsive to the strength of the wireless signal. Moreover, the antenna connection unit 6 transmits a control signal to change an operating mode such as a frame rate to the capsule endoscope apparatus 3 via the antenna apparatus 4 and the antenna cable 5.

The receiving apparatus 7 acquires the image data of the inside of the subject 2 based on the wireless signal transmitted from the capsule endoscope apparatus 3, via the antenna connection unit 6. The receiving apparatus 7 associates location information, time information indicating a time, and the like with the received image data and records the image data in a recording unit. The receiving apparatus 7 is carried by the subject 2, housed in the receiving apparatus holder while the capsule endoscope apparatus 3 is capturing images, for example, from the capsule endoscope apparatus 3 being introduced from the mouth of the subject 2 to passing through the digestive tract to being excreted from the subject 2. The receiving apparatus 7 is removed from the subject 2 after the end of the examination by the capsule endoscope apparatus 3, and is connected to the image processing apparatus 9 via the cradle 8 to transfer information such as the image data received from the capsule endoscope apparatus 3.

When the receiving apparatus 7 is attached, the cradle 8 acquires the image data and associated information associated with the image data, such as reception strength information, time information, and identification information of the capsule endoscope apparatus 3, from the recording unit of the receiving apparatus 7, and transfers the various pieces of acquired information to the image processing apparatus 9. Moreover, the cradle 8 outputs, to the receiving apparatus 7, an instruction signal input from the image processing apparatus 9.

The image processing apparatus 9 is configured using a workstation or personal computer including a display unit 91 such as an organic EL (Electro Luminescence) or liquid crystal display. The image processing apparatus 9 displays an image corresponding to the image data of the inside of the subject 2 acquired via the receiving apparatus 7. The image processing apparatus 9 includes an operation input device 92 such as a mouse 92a and a keyboard 92b. The operation input device 92 accepts input from a user.

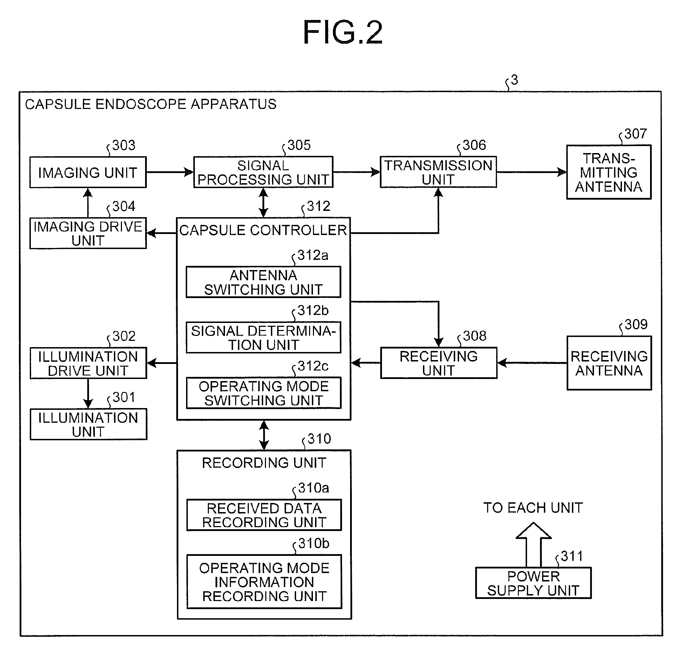

Next, the configuration of the capsule endoscope apparatus 3 illustrated in FIG. 1 will be described. FIG. 2 is a block diagram illustrating a general configuration of the capsule endoscope apparatus 3.

The capsule endoscope apparatus 3 illustrated in FIG. 2 includes an illumination unit 301, an illumination drive unit 302, an imaging unit 303, an imaging drive unit 304, a signal processing unit 305, a transmission unit 306, a transmitting antenna 307, a receiving unit 308, a receiving antenna 309, a recording unit 310, a power supply unit 311, and a capsule controller 312.

The illumination unit 301 is configured using a plurality of LEDs and the like, emits illumination light that illuminates the inside (body cavity) of the subject 2 including the visual field area of the imaging unit 303. The illumination drive unit 302 causes the illumination unit 301 to emit illumination light at specified brightness in specified cycles under the control of the capsule controller 312.

The imaging unit 303 is configured using an optical system including one or more lenses that concentrate reflected light of the illumination light emitted by the illumination unit 301, a CCD (Charge Coupled Device) or CMOS (Complementary Metal Oxide Semiconductor) that receives the light concentrated by the optical system on a light receiving surface and converts the light into an electric signal, and the like. The imaging unit 303 generates an image signal of the inside of the subject 2 (hereinafter referred to as "image data"). The imaging drive unit 304 causes the imaging unit 303 to output the image data (an analog signal) to the signal processing unit 305 at a specified timing under the control of the capsule controller 312.

The signal processing unit 305 performs specified signal processes such as a correlated double sampling process, an amplifying process, an A/D conversion process, and a multiplexing process on the image data input from the imaging unit 303 and accordingly generates image data corresponding to the imaging area inside the subject 2. The signal processing unit 305 outputs the image data to the transmission unit 306 under the control of the capsule controller 312.

The transmission unit 306 performs specified processes such as modulation and amplification on the image data input from the signal processing unit 305, and transmits the image data to the antenna apparatus 4 via the transmitting antenna 307 under the control of the capsule controller 312.

The receiving unit 308 performs specified processes such as demodulation and amplification on a control signal (wireless signal) received from the antenna apparatus 4 via the receiving antenna 309 and outputs the control signal to the capsule controller 312.

The recording unit 310 is configured using a volatile memory, non-volatile memory, or the like. The recording unit 310 includes a received data recording unit 310a that records received data corresponding to the control signal received by the receiving unit 308, and an operating mode information recording unit 310b that records various programs, such as an imaging program and an illumination program, for operating the capsule endoscope apparatus 3 in accordance with the operating mode of the capsule endoscope apparatus 3, various pieces of data to be used during the execution of each program, parameters necessary for the operation of signal processing by the signal processing unit 305, and the like. The operating mode here is a mode set such that the frame rate of image capture by the imaging unit 303, the transmission timing of the transmission unit 306, the pause timing of the imaging unit 303, the illumination timing, brightness, and a light control target value of the illumination unit 301, the illumination method of the illumination unit 301, and the like are respectively different.

The power supply unit 311 supplies power to each unit of the capsule endoscope apparatus 3 under the control of the capsule controller 312.

The capsule controller 312 is configured using a CPU (Central Processing Unit) and the like. The capsule controller 312 reads out various programs from the operating mode information recording unit 310b of the recording unit 310 to carry out calculations and accordingly performs things such as giving instructions to and transferring data to the units included in the capsule endoscope apparatus 3 to centrally control the operation of the capsule endoscope apparatus 3.

The detailed configuration of the capsule controller 312 will be described. The capsule controller 312 includes an antenna switching unit 312a, a signal determination unit 312b, and an operating mode switching unit 312c.

The antenna switching unit 312a activates the receiving unit 308 that receives a control signal from the outside in synchronization with a timing when the capsule endoscope apparatus 3 transmits a wireless signal including image data to the outside. Specifically, the antenna switching unit 312a activates the receiving unit 308 immediately after the transmission unit 306 transmits a wireless signal including image data of one frame via the transmitting antenna 307, and accordingly shifts to a state of being capable of receiving a control signal from the outside.

The signal determination unit 312b determines whether or not the contents of at least two or more control signals match among control signals transmitted respectively from a plurality of antennas of the antenna apparatus 4 and received by the receiving unit 308 a plurality of times during the reception period of the receiving unit 308. Specifically, the signal determination unit 312b determines whether or not the contents of a plurality of pieces of the received data recorded in the received data recording unit 310a of the recording unit 310 match each other.

The operating mode switching unit 312c switches the operating mode of the capsule endoscope apparatus 3 based on the determination result of the signal determination unit 312b. Specifically, if the signal determination unit 312b determines that the contents of at least two or more control signals match among control signals received a plurality of times by the receiving unit 308 during the reception period of the receiving unit 308, the operating mode switching unit 312c switches the operating mode of the capsule endoscope apparatus 3 to an operating mode corresponding to the control signal and causes the capsule endoscope apparatus 3 to operate in the operating mode only once.

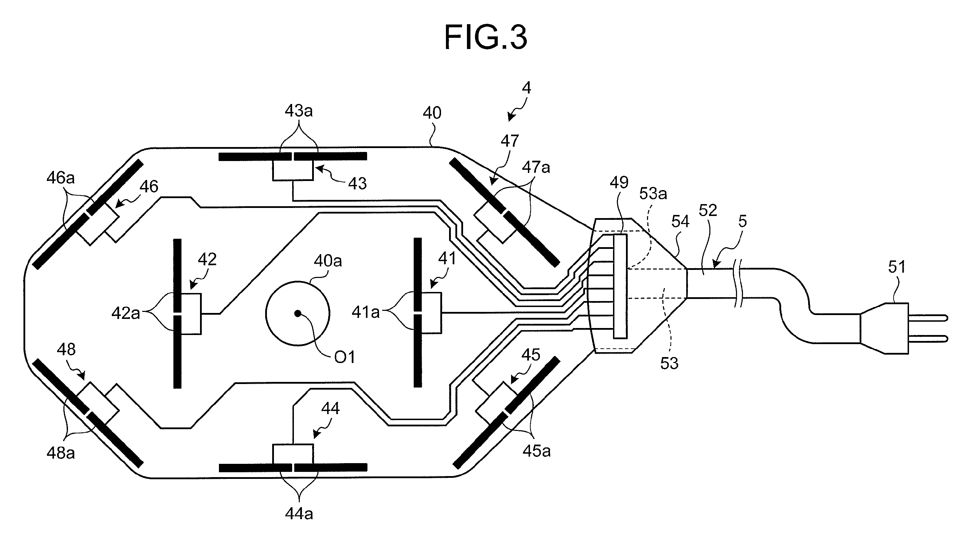

Next, a description is given of the detailed configurations of the antenna apparatus 4 and the antenna cable 5 illustrated in FIG. 1. FIG. 3 is a schematic diagram illustrating the configurations of the antenna apparatus 4 and the antenna cable 5.

The antenna apparatus 4 illustrated in FIG. 3 includes a polygonal sheet 40, a first antenna 41, a second antenna 42, a third antenna 43, a fourth antenna 44, a fifth antenna 45, a sixth antenna 46, a seventh antenna 47, an eighth antenna 48, and a connector section 49. The first antenna 41 to the eighth antenna 48 are respectively connected to the connector section 49, and provided on one polygonal sheet 40. In FIG. 3, a reference point 01 denotes the center of the polygonal sheet.

The polygonal sheet 40 is configured using a sheet-shaped flexible board. The principal surface of the polygonal sheet 40 forms a substantial octagon. The polygonal sheet 40 is formed in a size that covers the entire surface of the abdomen of the subject 2. The polygonal sheet 40 includes a positioning hole portion 40a forming a substantial circle.

The center of the positioning hole portion 40a is provided at a position that includes the reference point 01 of the polygonal sheet 40 at the center. The positioning hole portion 40a functions as a positioning portion that decides the position to attach the antenna apparatus 4 onto the subject 2 when the antenna apparatus 4 is attached to the subject 2. For example, if the polygonal sheet 40 is attached to the subject 2 such that an indicator region (for example, navel) on the body surface of the subject 2 is located at the center (the reference point 01) in the positioning hole portion 40a, the first antenna 41 to the eighth antenna 48 of the antenna apparatus 4 are attached correctly to specified attachment positions on the body surface of the subject 2. The principal surface of the polygonal sheet 40 is not necessarily a substantial octagon but may be, for example, a square.

Moreover, in the polygonal sheet 40, a cover portion 54 that covers a connection portion 53a between the polygonal sheet 40 and a proximal end 53 of the antenna cable 5 is made of an elastic member that progressively becomes thin from the proximal end 53 toward the polygonal sheet 40 in order to prevent the polygonal sheet 40 from being bent at an edge of the cover portion 54. The antenna apparatus 4 is inserted into the antenna holder and attached to the subject 2 during the examination. However, the antenna apparatus 4 and the antenna holder are formed in an up-down and/or left-right asymmetric shapes. Accordingly, the antenna apparatus 4 cannot be put into the antenna holder when being upside down or front side back so that it is possible to prevent the antenna apparatus 4 from being attached to the subject 2 in a wrong orientation. Moreover, there may be a marking, on the surface of the antenna apparatus 4, to avoid confusing the up side with the down side and the front side with the back side of the antennas.

The first antenna 41 and the second antenna 42 are respectively arranged at positions facing each other across the reference point 01 of the polygonal sheet 40. The first antenna 41 and the second antenna 42 are respectively an equal distance away from the reference point 01. The first antenna 41 and the second antenna 42 respectively include an element portion 41a and an element portion 42a formed by printed wiring, on the polygonal sheet 40. The first antenna 41 and the second antenna 42 are connected by flat transmission lines (striplines) to the connector section 49 provided to the polygonal sheet 40.

The third antenna 43 and the fourth antenna 44 are arranged at positions respectively rotated by 90 degrees around the reference point 01 in a plane with respect to a straight line linking the center of gravity of the first antenna 41 and the center of gravity of the second antenna 42. The third antenna 43 and the fourth antenna 44 are respectively an equal distance away from the reference point 01. The third antenna 43 and the fourth antenna 44 respectively include an element portion 43a and an element portion 44a formed by printed wiring, on the polygonal sheet 40. The third antenna 43 and the fourth antenna 44 are connected to the connector section 49 by flat transmission lines.

The fifth antenna 45 and the sixth antenna 46 are respectively arranged at positions where their extension directions form 45 degrees with the straight line linking the center of gravity of the first antenna 41 and the center of gravity of the second antenna 42, and at positions where each center of gravity is in the plane. The fifth antenna 45 and the sixth antenna 46 are respectively arranged at positions on an outer peripheral side in the plane than the first antenna 41 and the second antenna 42. The fifth antenna 45 and the sixth antenna 46 respectively include an element portion 45a and an element portion 46a formed by printed wiring, on the polygonal sheet 40. The fifth antenna 45 and the sixth antenna 46 are connected to the connector section 49 by flat transmission lines.

The seventh antenna 47 and the eighth antenna 48 are respectively arranged at positions where their extension directions form 45 degrees with the straight line linking the center of gravity of the first antenna 41 and the center of gravity of the second antenna 42, and a straight line linking the center of gravity of the third antenna 43 and the center of gravity of the fourth antenna 44, and at position where each center of gravity is in the plane. The seventh antenna 47 and the eighth antenna 48 are respectively arranged at positions on an outer peripheral side in the plane than the first antenna 41 and the second antenna 42. The seventh antenna 47 and the eighth antenna 48 respectively include an element portion 47a and an element portion 48a formed by printed wiring, on the polygonal sheet 40. The seventh antenna 47 and the eighth antenna 48 are connected to the connector section 49 by flat transmission lines.

The antenna cable 5 propagates wireless signals received respectively by the first antenna 41 to the eighth antenna 48 to the antenna connection unit 6, and propagates a control signal to communicate wirelessly with the capsule endoscope apparatus 3, the control signal having been input from the antenna connection unit 6, to the first antenna 41 to the eighth antenna 48. The antenna cable 5 includes a bending prevention portion 51, a flexible portion 52, and the proximal end 53. The bending prevention portion 51 is connected to the antenna connection unit 6. The flexible portion 52 includes core wires, the number of which is proportional to the number of the first antenna 41 to the eighth antenna 48. The proximal end 53 is connected to the connector section 49 at a position a specified distance away from the straight line passing through the reference point 01.

In the antenna apparatus 4 having the above-mentioned configuration, the first antenna 41 to the eighth antenna 48 are arranged with respect to a region to serve as an indicator on the body surface of the subject 2, and accordingly relative positions of the antennas to the organs in the subject 2, which are the lumen in the body through which the capsule endoscope apparatus 3 passes, can be arranged with high accuracy. Consequently, with simple operation of attaching the antenna apparatus 4 to the subject 2 using the positioning hole portion 40a, the position of the antenna apparatus 4 with respect to the subject 2 can be easily determined. A transparent member, for example, a transparent vinyl sheet, may be provided to the positioning hole portion 40a.

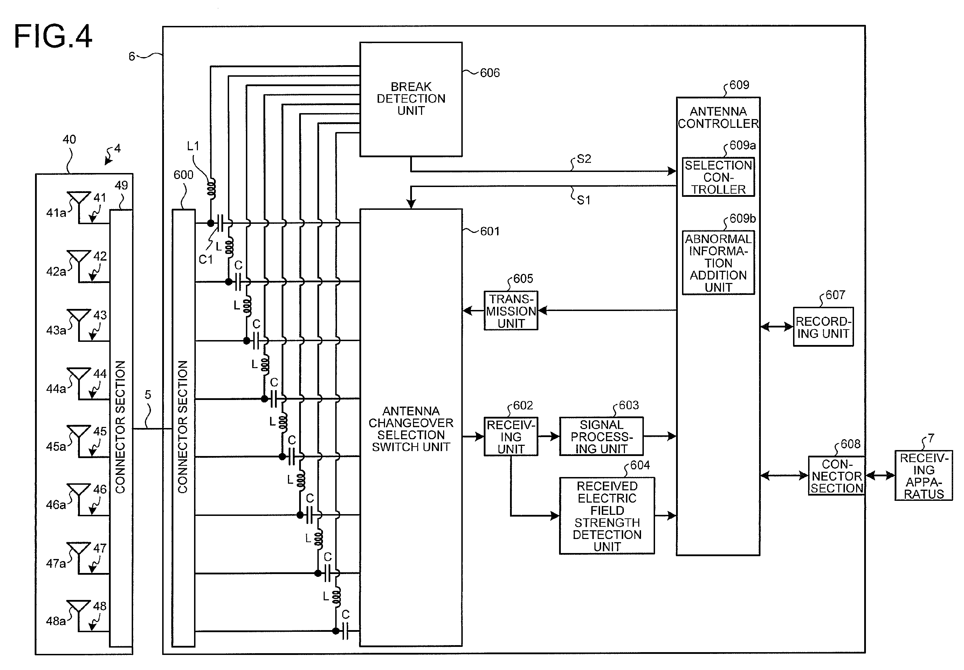

Next, a description is given of the functions of the antenna connection unit 6 described in FIG. 1 and the antenna apparatus 4 described in FIG. 3. FIG. 4 is a block diagram illustrating functional configurations of the antenna apparatus 4 and the antenna connection unit 6. When any of the first antenna 41 to the eighth antenna 48 is indicated in the following description, it is simply described as the first antenna 41.

The antenna connection unit 6 illustrated in FIG. 4 includes a connector section 600, an antenna changeover selection switch unit 601, a receiving unit 602, a signal processing unit 603, a received electric field strength detection unit 604, a transmission unit 605, a break detection unit 606, a recording unit 607, a connector section 608, and an antenna controller 609.

The connector section 600 is detachably connected to the bending prevention portion 51 of the antenna cable 5. The connector section 600 is electrically connected to each of the antenna changeover selection switch unit 601 and the break detection unit 606.

The antenna changeover selection switch unit 601 is configured using a mechanical switch, semiconductor switch, or the like. The antenna changeover selection switch unit 601 is electrically connected to each of the first antenna 41 to the eighth antenna 48 via a capacitor C1. If a switching signal S1 to switch antennas that receive a wireless signal is input from the antenna controller 609 via the receiving apparatus 7, the antenna changeover selection switch unit 601 selects the first antenna 41 instructed by the switching signal S1, and electrically connects the selected first antenna 41 and the receiving unit 602. Moreover, if the switching signal S1 to switch antennas that transmit a control signal is input from the antenna controller 609 via the receiving apparatus 7, the antenna changeover selection switch unit 601 selects the first antenna 41 instructed by the switching signal S1, and electrically connects the selected first antenna 41 and the transmission unit 605. The capacities of the capacitors connected respectively to the first antenna 41 to the eighth antenna 48 are equal to the capacity of the capacitor C1. In the first embodiment, the antenna changeover selection switch unit 601 functions as an antenna selector.

The receiving unit 602 performs specified processes such as demodulation and amplification on a wireless signal received by the antenna changeover selection switch unit 601 via the selected first antenna 41, and outputs the wireless signal to each of the signal processing unit 603 and the received electric field strength detection unit 604.

The signal processing unit 603 extracts image data from the wireless signal input from the receiving unit 602, performs specified processes, for example, various image processing and an A/D conversion process, on the extracted image data, and outputs the image data to the antenna controller 609. Specifically, the signal processing unit 603 performs an amplification process, a noise reduction process, and the like on the image data, and then performs A/D conversion on the image data to output to the antenna controller 609.

The received electric field strength detection unit 604 detects reception strength proportional to the strength of the wireless signal input from the receiving unit 602, and outputs, to the antenna controller 609, a received strength signal (RSSI: Received Signal Strength Indicator) corresponding to the detected reception strength.

The transmission unit 605 performs specified processes such as modulation and amplification on a control signal input from the receiving apparatus 7 via the connector section 608 and the antenna controller 609, and transmits a wireless signal to the capsule endoscope apparatus 3 via the first antenna 41 selected by the antenna changeover selection switch unit 601.

The break detection unit 606 is electrically connected to each of the first antenna 41 to the eighth antenna 48 respectively via a coil L1. If the break detection unit 606 detects a break in the first antenna 41 selected by the antenna changeover selection switch unit 601 and an abnormality occurs in the first antenna 41, the break detection unit 606 outputs, to the antenna controller 609, an abnormal signal S2 indicating that a break occurs in the first antenna 41. For example, the break detection unit 606 detects based on the voltage of the first antenna 41 whether or not a break occurs in the first antenna 41. The break detection unit 606 may detect an abnormality of a short circuit of the first antenna 41. Furthermore, the electrical characteristics of coils respectively connected to the first antenna 41 to the eighth antenna 48 are equal to those of the coil L1. In the first embodiment, the break detection unit 606 functions as a break determination unit.

The recording unit 607 is configured using a semiconductor memory such as a flash memory or RAM. Image data captured by the capsule endoscope apparatus 3, various pieces of information associated with the image data, for example, location information of the capsule endoscope apparatus 3, reception strength information, and identification information for identifying an antenna that has received a wireless signal, various programs to be executed by the antenna connection unit 6, and the like are recorded in the recording unit 607.

The connector section 608 has a function as a communication interface, and performs transmission and reception bi-directionally to and from the receiving apparatus 7. Moreover, power is supplied from the receiving apparatus 7 to each unit of the antenna connection unit 6 via the connector section 608.

The antenna controller 609 is configured using a CPU and the like. The antenna controller 609 reads out a program from the recording unit 607 and executes the program, and performs things such as giving instructions to and transferring data to the units included in the antenna connection unit 6 to centrally controls the operation of the antenna connection unit 6.

A detailed configuration of the antenna controller 609 will be described. The antenna controller 609 includes a selection controller 609a and an abnormal information addition unit 609b.

The selection controller 609a selects an antenna that receives a wireless signal transmitted from the capsule endoscope apparatus 3. Specifically, the selection controller 609a selects an antenna that receives a wireless signal transmitted from the capsule endoscope apparatus 3 based on the reception strength (input power) of each of the first antenna 41 to the eighth antenna 48, the reception strength having been detected by the received electric field strength detection unit 604. For example, the selection controller 609a drives the antenna changeover selection switch unit 601 at every specified timing, for example, at intervals of 100 msec, sequentially selects the first antenna 41 to the eighth antenna 48 to receive wireless signals, and repeats this process until the reception strength detected by the received electric field strength detection unit 604 reaches a specified value. Moreover, the selection controller 609a selects an antenna that transmits a control signal input from the receiving apparatus 7. Specifically, the selection controller 609a sequentially switches and selects the first antenna 41 to the eighth antenna 48 at every specified timing to transmit a control signal indicating a control signal with the same content.

If the break detection unit 606 detects a break in any of the first antenna 41 to the eighth antenna 48, the abnormal information addition unit 609b adds break information indicating that a break occurs in any of the first antenna 41 to the eighth antenna 48 to wireless signals received respectively by the first antenna 41 to the eighth antenna 48. Specifically, the abnormal information addition unit 609b adds a flag indicating abnormal information to the image data that the signal processing unit 603 has performed signal processing on wireless signals received respectively by the first antenna 41 to the eighth antenna 48.

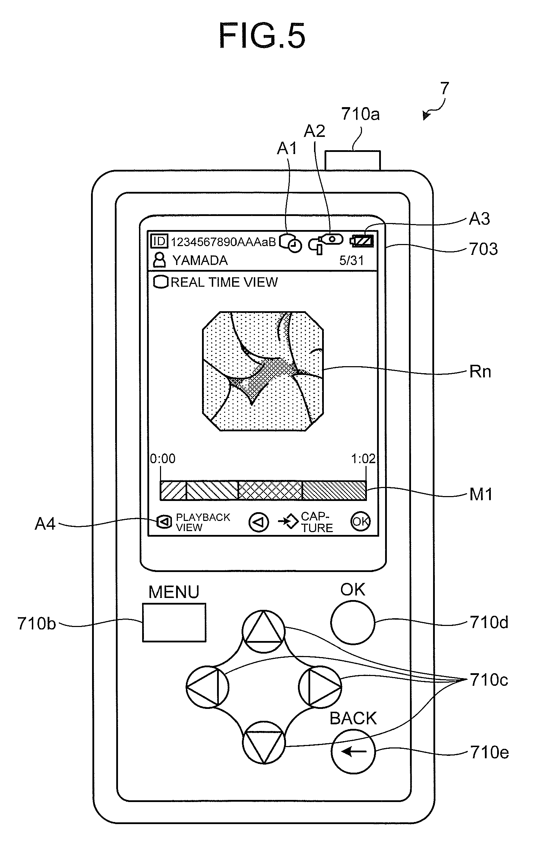

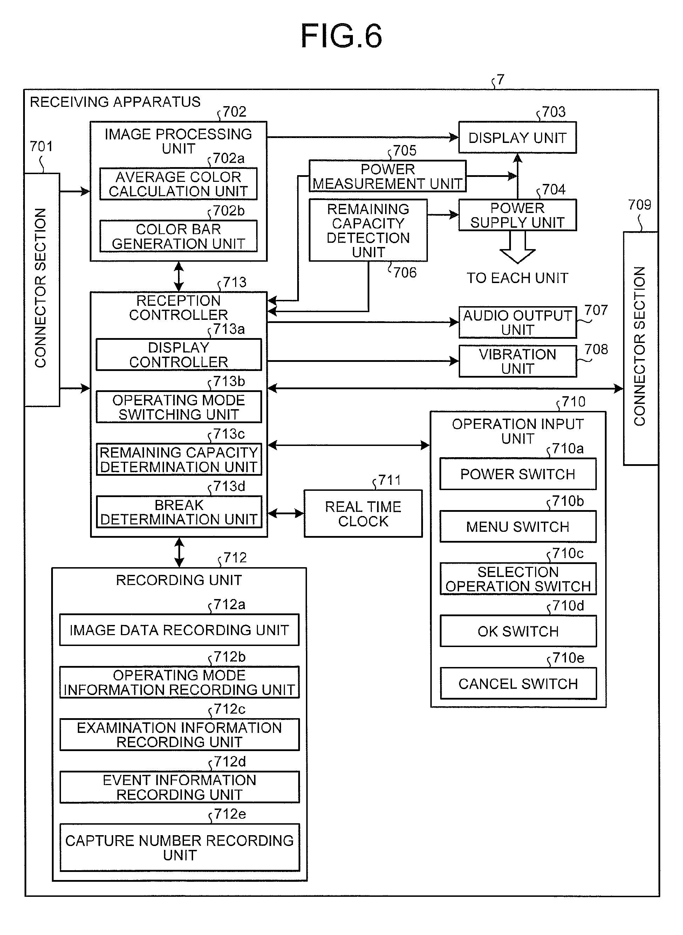

Next, the receiving apparatus 7 illustrated in FIG. 1 will be described. FIG. 5 is a schematic diagram illustrating the appearance of the receiving apparatus 7. FIG. 6 is a block diagram illustrating a functional configuration of the receiving apparatus 7.

The receiving apparatus 7 illustrated in FIGS. 5 and 6 includes a connector section 701, an image processing unit 702, a display unit 703, a power supply unit 704, a power measurement unit 705, a remaining capacity detection unit 706, an audio output unit 707, a vibration unit 708, a connector section 709, an operation input unit 710, a real time clock 711, a recording unit 712, and a reception controller 713.

The connector section 701 has a function as a communication interface, and performs transmission and reception bi-directionally to and from the antenna connection unit 6. Moreover, the connector section 701 supplies the power supplied from the power supply unit 704 to the antenna connection unit 6.

The image processing unit 702 is realized by an image engine using an FPGA (Field Programmable Gate Array) and the like. The image processing unit 702 performs specified image processing on image data (RAW data) input from the antenna connection unit 6 via the connector section 701, and outputs the image data to the recording unit 712 via the reception controller 713. Specifically, the image processing unit 702 performs, on the image data, image processing including at least an optical black subtraction process, a white balance adjustment process, a demosaicing (developing process), a binarization process (halftoning process) by error diffusion, color conversion, gray scale transformation (gamma conversion and the like), smoothing (noise reduction and the like), image sharpening (edge enhancement and the like), and accordingly generates image data for display and recording. The image processing unit 702 outputs the image data for display to the display unit 703. Moreover, the image processing unit 702 includes an average color calculation unit 702a and a color bar generation unit 702b.

The average color calculation unit 702a calculates the average color of an image based on image data input from the antenna connection unit 6 via the connector section 701. Specifically, the average color calculation unit 702a extracts data corresponding to pixel values at a plurality of (for example, four) specified locations in the image, and calculates the average color of the image based on the extracted data. The average color calculation unit 702a performs the process whenever image data is input.

The color bar generation unit 702b generates a color bar to display on the display unit 703 based on the average color calculated by the average color calculation unit 702a.

The display unit 703 is configured using a liquid crystal or organic EL (Electro Luminescence) display panel, or the like. As illustrated in FIG. 5, the display unit 703 displays an image Rₙ (n = natural number) corresponding to image data transmitted from the capsule endoscope apparatus 3, patient ID, patient name, examination date and time, time, an LCD icon A1 indicating the state of the display unit 703, an antenna icon A2 indicating the state of the antenna apparatus 4, a remaining capacity icon A3 indicating the remaining capacity of the power supply unit 704, operating mode information indicating a current operating mode of the receiving apparatus 7, an operating mode icon A4 that accepts the input of an instruction signal to instruct a change of the operating mode of the receiving apparatus 7, a color bar M1, and various icons. The image displays include a real time view display to display a live view image corresponding to image data sequentially transmitted from the capsule endoscope apparatus 3 in chronological order, a playback view display to play back image data recorded in the recording unit 712, a capture image display to play back capture image data, and the like.

Moreover, the color bar M1 is a band-shaped image where the average color of each image of the inside of the subject 2 captured by the capsule endoscope apparatus 3 is displayed along a time axis. The overall length of the color bar M1 corresponds to time measured from the time of the start of an examination (at power-up of the capsule endoscope apparatus 3) to the time of capturing the latest image. The user refers to the color bar M1 and accordingly can distinguish the kind of a region (organ) corresponding to the average color of each captured image and grasp the elapsed time of the examination.

The power supply unit 704 is configured using a lithium battery and the like, and supplies power to each unit of the receiving apparatus 7 including the display unit 703. Moreover, the power supply unit 704 supplies power to the antenna connection unit 6 connected via the connector section 701.

The power measurement unit 705 measures the amount of power supplied to the display unit 703 by the power supply unit 704 and outputs the measurement result to the reception controller 713.

The remaining capacity detection unit 706 is configured using a remaining battery level measurement IC and the like, detects the remaining capacity (amount of power) stored in the power supply unit 704 at the start of the examination of the subject 2, and outputs the detected result to the reception controller 713.

The audio output unit 707 is configured using a speaker and the like, and outputs audio to the outside under the control of the reception controller 713.

The vibration unit 708 is configured using a motor and the like, and is driven to vibrate the receiving apparatus 7 under the control of the reception controller 713.

The connector section 709 has a function as a communication interface, and performs transmission and reception bi-directionally to and from the image processing apparatus 9 via the cradle 8.

The operation input unit 710 includes a power switch 710a that switches the power state of the receiving apparatus 7 between an on state and an off state, a menu switch 710b that accepts the input of an instruction signal to display a menu screen on the display unit 703, a selection operation switch 710c that accepts the input of an instruction signal to switch the selection setting of the receiving apparatus 7 on the menu screen or the like, an OK switch 710d that decides an operation on the menu screen or the like, and a cancel switch 710e that cancels an operation on the menu screen or the like. A touch panel, superimposed on the display screen of the display unit 703, for accepting the input of a location signal according to a contact position from the outside may be provided as the operation input unit 710.

The real time clock 711 functions as a timer of the receiving apparatus 7, and outputs time information to the reception controller 713. Moreover, the real time clock 711 synchronizes with the time information of a real time clock (not illustrated) of the image processing apparatus 9 when the receiving apparatus 7 is connected to the image processing apparatus 9 via the cradle 8.

The recording unit 712 is configured using an SDRAM (Synchronous Dynamic Random Access Memory), a flash memory, or the like. The recording unit 712 includes an image data recording unit 712a that records image data captured by the capsule endoscope apparatus 3, an operating mode information recording unit 712b that records operating mode information on an operating mode in which the capsule endoscope apparatus 3 operates, an examination information recording unit 712c that records subject information on the subject 2, an event information recording unit 712d that records event information upon the guidance of an event set for the receiving apparatus 7, and a capture number recording unit 712e that records a record number of a capture image.

The reception controller 713 is configured using a CPU and the like. The reception controller 713 reads out a program from the recording unit 712 and executes the program, and performs things such as giving instructions to and transferring data to the units included in the receiving apparatus 7 to centrally control the operation of the receiving apparatus 7.

The detailed configuration of the reception controller 713 will be described. The reception controller 713 includes a display controller 713a, an operating mode switching unit 713b, a remaining capacity determination unit 713c, and a break determination unit 713d.

The display controller 713a controls a display mode of the display unit 703. Specifically, the display controller 713a causes the display unit 703 to display an image corresponding to image data on which the image processing unit 702 has performed image processing, and the color bar M1 in response to an instruction signal input from the operation input unit 710. Moreover, the display controller 713a causes the display unit 703 to display the menu screen if an instruction signal to instruct the display of the menu screen is input from the menu switch 710b.

The operating mode switching unit 713b switches the antenna apparatus 4 from a state of being capable of receiving a wireless signal to a state of being capable of transmitting a wireless signal, via the antenna controller 609 of the antenna connection unit 6 at every specified timing. Specifically, the operating mode switching unit 713b receives a wireless signal from the capsule endoscope apparatus 3, then controls the antenna controller 609 of the antenna connection unit 6, and accordingly switches the antenna apparatus 4 to the state of being capable of transmitting a control signal to cause the first antenna 41 to the eighth antenna 48 of the antenna apparatus 4 to sequentially transmit a control signal having the same instruction content at every specified timing. For example, after the first antenna 41 receives a wireless signal from the capsule endoscope apparatus 3, the operating mode switching unit 713b outputs, to the selection controller 609a, an instruction signal to connect the first antenna 41 and the transmission unit 605 to the antenna controller 609, and accordingly switches the first antenna 41 from the receiving antenna to the transmitting antenna to transmit a control signal to the transmission unit 605. The operating mode switching unit 713b may set the first antenna 41 to the fourth antenna 44 as the receiving antennas to perform reception from the capsule endoscope apparatus 3, and switch the fifth antenna 45 to the eighth antenna 48 to the transmitting antenna to transmit a control signal.

The remaining capacity determination unit 713c determines whether or not a remaining capacity obtained by subtracting the power consumption measured by the power measurement unit 705 from the remaining capacity stored in the power supply unit 704 and detected by the remaining capacity detection unit 706 immediately after the power-up of the receiving apparatus 7 is equal to or less than a preset threshold value.

The break determination unit 713d determines whether or not information indicating that a break occurs in one or more of the first antenna 41 to the eighth antenna 48 has been added to image data input from the antenna connection unit 6 and accordingly determines whether or not a break occurs in the antenna apparatus 4 or the antenna cable 5.

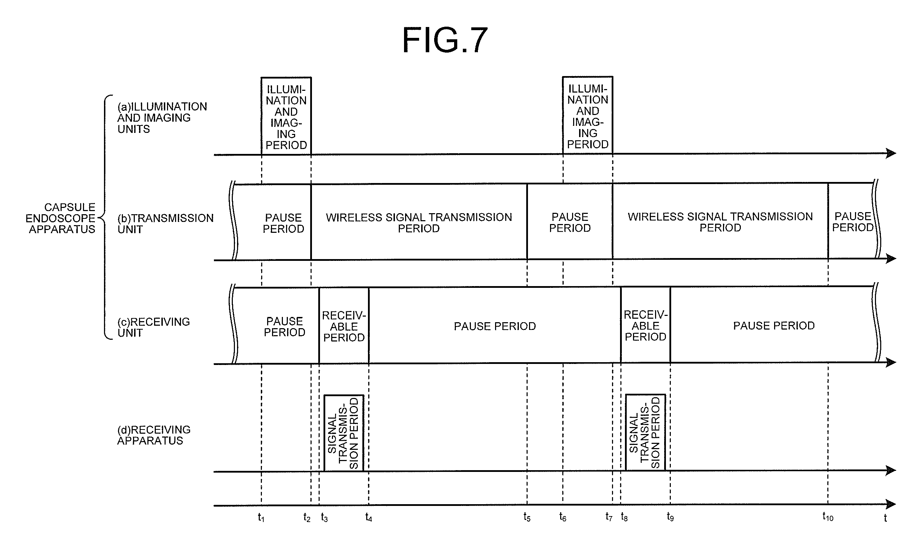

A description is given of timings of wireless signals transmitted bi-directionally between the capsule endoscope apparatus 3 and the receiving apparatus 7 in the capsule endoscope system 1 having the above-mentioned configuration. FIG. 7 is a timing chart illustrating the relationship between transmission timings when the capsule endoscope apparatus 3 transmits image data and transmission timings when the receiving apparatus 7 transmits a control signal. In FIG. 7, the horizontal axis indicates time. Moreover, FIG. 7(a) illustrates the operation timings of the imaging unit 303 and the illumination unit 301 of the capsule endoscope apparatus 3. FIG. 7(b) illustrates the transmission timings when the transmission unit 306 of the capsule endoscope apparatus 3 transmits image data. FIG. 7(c) illustrates the activation timings when the receiving unit 308 of the capsule endoscope apparatus 3 can receive a wireless signal. FIG. 7(d) illustrates the transmission timings when the receiving apparatus 7 transmits a control signal.

As illustrated in FIG. 7, after the illumination and imaging operations are complete (time t₁ to time t₂ and time t₆ to time t₇), the capsule controller 312 activates the receiving unit 308 and causes the receiving unit 308 to shift from a pause state where the receiving unit 308 pauses to the receiving state where the receiving unit 308 can receive control signals from the receiving apparatus 7 (time t₃ to time t₄ and time t₈ to time t₉) in a wireless signal transmission period (time t₂ to time t₅ and time t₇ to time t₁₀) from immediately after causing the transmission unit 306 to start transmitting wireless signals via the transmitting antenna 307 to the completion of the transmission of the wireless signals.

In the period during which the capsule endoscope apparatus 3 can receive control signals, the receiving apparatus 7 causes the transmission unit 605 to transmit a control signal to the capsule endoscope apparatus 3 via any of the first antenna 41 to the eighth antenna 48 when having received a wireless signal from the capsule endoscope apparatus 3. Furthermore, the receiving apparatus 7 transmits a control signal including the operating mode of the same content a plurality of (for example, three or more) times during the receivable period of the capsule endoscope apparatus 3 (time t₃ to time t₄ and time t₈ to time t₉) while switching sequentially between an antenna to transmit a control signal and an antenna to receive a wireless signal via the antenna connection unit 6 at every specified timing.

Consequently, the capsule endoscope apparatus 3 does not need to always supply power to the receiving unit 308. Accordingly, the power consumption of the capsule endoscope apparatus 3 can be reduced. Furthermore, the capsule endoscope apparatus 3 activates the transmission unit 306 only in the transmission period to transmit a wireless signal as image data. Accordingly, the power consumption of the capsule endoscope apparatus 3 can be further reduced. As a consequence, it is possible to reduce the capacity of the power supply unit 311 such as a battery to be mounted in the capsule endoscope apparatus 3 so that the size of the capsule endoscope apparatus 3 can be further reduced.

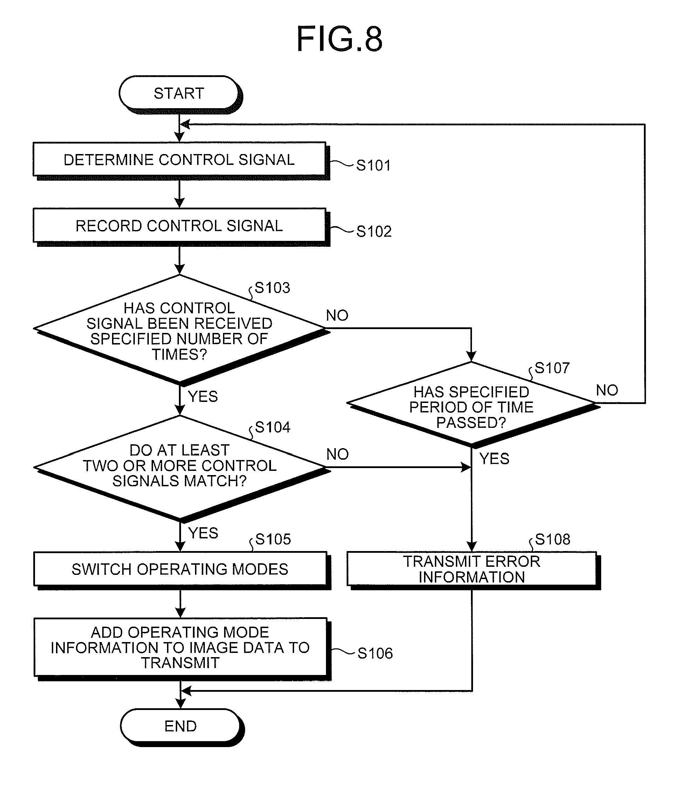

A description is given of a process to integrate control signals received a plurality of times from the receiving apparatus 7 by the capsule endoscope apparatus 3. FIG. 8 is a flowchart illustrating an outline of the process to be executed by the capsule endoscope apparatus 3, and illustrates an outline of the process of when the capsule endoscope apparatus 3 receives a plurality of control signals from the receiving apparatus 7 in the receivable period (time t₃ to time t₄ and time t₈ to time t₉).

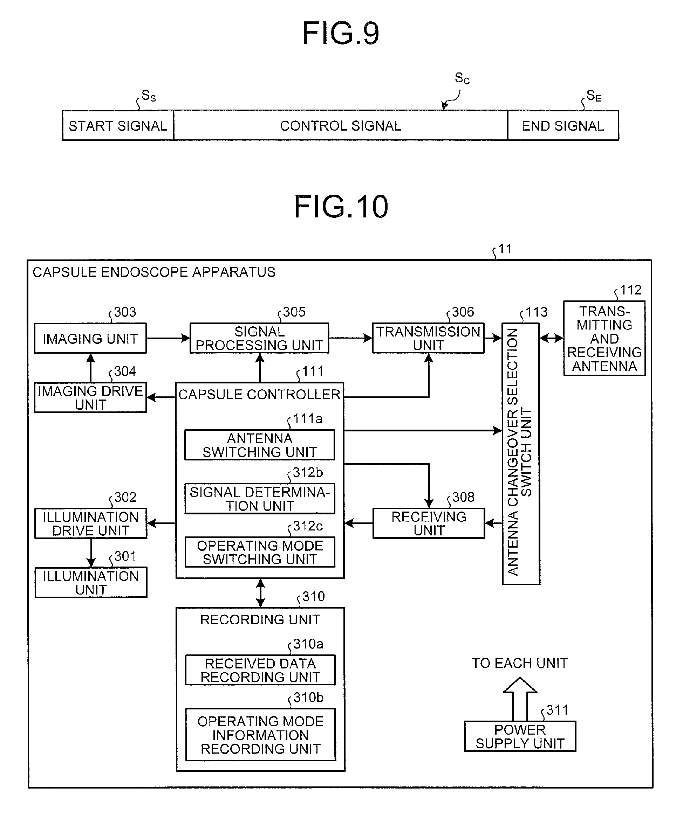

As illustrated in FIG. 8, the signal determination unit 312b determines whether or not the receiving unit 308 has received a control signal from the receiving apparatus 7 (Step S101). Specifically, as illustrated in FIG. 9, the signal determination unit 312b determines whether or not a start signal S_{S} and an end signal S_{E} are included in a control signal S_{C} input from the receiving unit 308. At this point in time, if the start signal S_{S} and the end signal S_{E} cannot be extracted from the control signal S_{C}, the signal determination unit 312b add, to the received control signal S_{C}, information to the effect of failing in reception from an antenna that has transmitted the control signal S_{C}.

Next, the capsule controller 312 records the control signal received by the receiving unit 308 in the received data recording unit 310a of the recording unit 310 (Step S102).

If the receiving unit 308 subsequently receives a control signal from the receiving apparatus 7 a specified number of times (Step S103: Yes), the signal determination unit 312b determines whether or not at least two or more control signals match among the plurality of control signals recorded in the received data recording unit 310a of the recording unit 310 (Step S104). Specifically, the signal determination unit 312b determines whether or not at least two or more instruction contents, for example, operating modes, match among the plurality of control signals recorded in the received data recording unit 310a. If the signal determination unit 312b determines that at least two or more control signals match (Step S104: Yes), the capsule endoscope apparatus 3 proceeds to Step S105.

Next, the operating mode switching unit 312c integrates the plurality of control signals recorded in the received data recording unit 310a of the recording unit 310 as one control signal, and switches the operating modes of the capsule endoscope apparatus 3 based on the integrated control signal and the operating modes recorded in the operating mode information recording unit 310b (Step S105).

The capsule controller 312 then adds current operating mode information of the capsule endoscope apparatus 3 to image data and causes the transmission unit 306 to transmit the image data (Step S106). The capsule endoscope apparatus 3 subsequently ends the process.

In Step S103, if the receiving unit 308 has not received a control signal from the receiving apparatus 7 the specified number of times (Step S130: No), the capsule controller 312 judges whether or not a specified period of time has passed since the first reception time of the control signal (Step S107). If the capsule controller 312 judges that the specified period of time has passed (Step S107: Yes), the capsule endoscope apparatus 3 proceeds to Step S108 described below. In contrast, if the capsule controller 312 judges that the specified period of time has not passed (Step S107: No), the capsule endoscope apparatus 3 returns to Step S101.

In Step S104, if the signal determination unit 312b determines that at least two or more control signals do not match (Step S104: No), the capsule endoscope apparatus 3 proceeds to Step S108.

Next, the capsule controller 312 adds, to image data, error information indicating failure in the reception of a control signal from the receiving apparatus 7, and causes the transmission unit 306 to transmit the image data (Step S108). The capsule endoscope apparatus 3 subsequently ends the process.

According to the first embodiment described above, the capsule controller 312 activates the receiving unit 308 in synchronization with a transmission timing when the transmission unit 306 transmits a wireless signal. Accordingly, the consumption of the power supply unit 311 can be reduced.

Moreover, in the first embodiment, an error correction code may be added to a wireless signal to be transmitted by the receiving apparatus 7 to transmit to the capsule endoscope apparatus 3. Furthermore, a control signal having the same instruction content may be transmitted on the same antenna a plurality of times.

Moreover, in the first embodiment, the first antenna 41 to the eighth antenna 48 are respectively capable of transmission and reception. However, a transmitting antenna and a receiving antenna may be provided separately.

Moreover, in the first embodiment, the antenna apparatus 4, the antenna cable 5, the antenna connection unit 6, and the receiving apparatus 7 may be integrally formed. Furthermore, the antenna apparatus 4, the antenna cable 5, and the antenna connection unit 6 may be integrally formed.

Moreover, in the first embodiment, the operating mode switching unit 713b switches sequentially among the first antenna 41 to the eighth antenna 48 at specified intervals, and sequentially transmits control signals having the same instruction content, but may, for example, sequentially transmit only strength signals included in the wireless signals received from the capsule endoscope apparatus 3. In this case, the capsule endoscope apparatus 3 may transmit information on an antenna having the strongest reception strength to the receiving apparatus 7, and the receiving apparatus 7 may select the antenna having the strongest reception strength based on the information transmitted from the capsule endoscope apparatus 3, and transmit a control signal to the capsule endoscope apparatus 3 via the selected antenna. Consequently, it is possible to reduce the data amount of data to be transmitted from the receiving apparatus 7 to the capsule endoscope apparatus 3.

Moreover, in the first embodiment, the capsule controller 312 activates the receiving unit 308 whenever causing the transmission unit 306 to transmit a wireless signal. However, the receiving unit 308 may be activated, for example, after the transmission unit 306 transmits a wireless signal a specified number of times, for example, four times.

### (Second Embodiment)

Next, a second embodiment of the present invention will be described. In the above-mentioned first embodiment, the capsule endoscope apparatus includes the transmitting and receiving antennas. In the second embodiment, however, wireless signals are transmitted and received on one antenna. Furthermore, the relationship between the transmission timing when the capsule endoscope apparatus transmits a wireless signal and the transmission timing when the receiving apparatus transmits a control signal is different from the above-mentioned first embodiment. Hence, in the following, after the configuration of the capsule endoscope apparatus is described, the relationship between the transmission timing when the capsule endoscope apparatus transmits a wireless signal and the transmission timing when the receiving apparatus transmits a control signal will be described. The same reference numerals will be used to refer to the same elements as those of the above-mentioned first embodiment.

FIG. 10 is a block diagram illustrating a functional configuration of a capsule endoscope apparatus according to the second embodiment.

A capsule endoscope apparatus 11 illustrated in FIG. 10 includes the illumination unit 301, the illumination drive unit 302, the imaging unit 303, the imaging drive unit 304, the signal processing unit 305, the transmission unit 306, the receiving unit 308, the recording unit 310, the power supply unit 311, a transmitting and receiving antenna 112, an antenna changeover selection switch unit 113, and a capsule controller 111.

The antenna changeover switch unit 113 is configured using a mechanical switch, semiconductor switch, or the like, and electrically connects the transmitting and receiving antenna 112 to the transmission unit 306 or the receiving unit 308 under the control of the capsule controller 111.

The capsule controller 111 is configured using a CPU and the like. The capsule controller 111 reads out various programs from the recording unit 310 and carries out calculations, and accordingly performs things such as giving instructions to and transferring data to the units included in the capsule endoscope apparatus 11 to centrally control the operation of the capsule endoscope apparatus 11.

The detailed configuration of the capsule controller 111 will be described. The capsule controller 111 includes an antenna switching unit 111a, the signal determination unit 312b, and the operating mode switching unit 312c.

The antenna switching unit 111a drives the receiving unit 308, and causes the receiving unit 308 to shift to the state of being capable of receiving a control signal from the receiving apparatus 7 via the transmitting and receiving antenna 112 in a pause period from the time when the transmission unit 306 completes transmission by transmitting a wireless signal including image data of one frame to the receiving apparatus 7 via the transmitting and receiving antenna 112, to the start of the transmission of a wireless signal including image data of the next one frame.

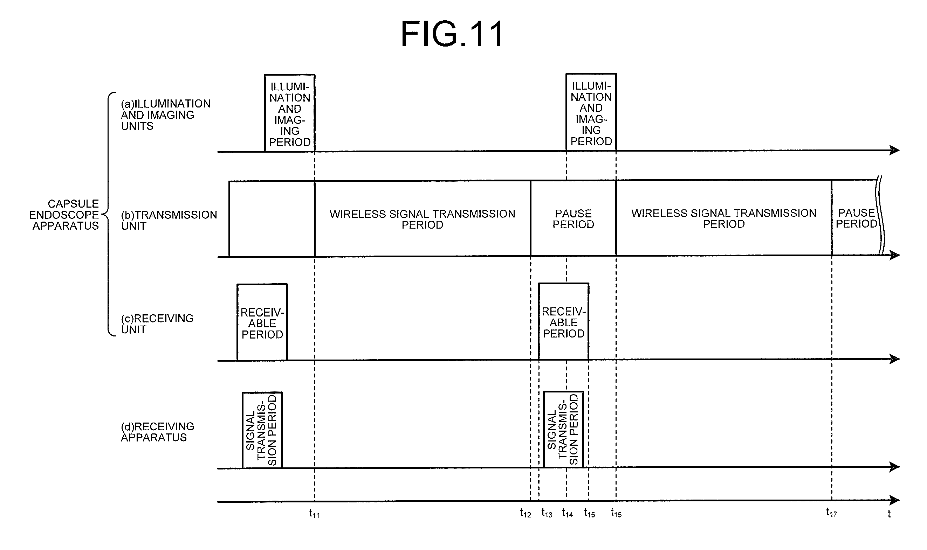

A description is given of timings of wireless signals transmitted bi-directionally between the capsule endoscope apparatus 11 having the above-mentioned configuration and the receiving apparatus 7. FIG. 11 is a timing chart illustrating the relationship between transmission timings when the capsule endoscope apparatus 11 transmits a wireless signal including image data and transmission timings when the receiving apparatus 7 transmits a control signal. In FIG. 11, the horizontal axis indicates time. Moreover, FIG. 11(a) illustrates the operation timings of the imaging unit 303 and the illumination unit 301 of the capsule endoscope apparatus 11. FIG. 11(b) illustrates the transmission timings when the transmission unit 306 of the capsule endoscope apparatus 11 transmits image data. FIG. 11(c) illustrates the activation timings when the receiving unit 308 of the capsule endoscope apparatus 11 can receive a wireless signal. FIG. 11(d) illustrates the transmission timings when the receiving apparatus 7 transmits a control signal.

As illustrated in FIG. 11, after the illumination and imaging period for both the illumination unit 301 and the imaging unit 303 is complete (time t₁₁), the capsule controller 111 drives the antenna changeover switch unit 113, connects the transmission unit 306 and the transmitting and receiving antenna 112, and causes the transmission unit 306 to transmit a wireless signal including image data captured by the imaging unit 303 (time t₁₁ to time t₁₂ and time t₁₆ to time t₁₇).

Next, after the transmission unit 306 completes transmitting the wireless signal including image data, the capsule controller 111 causes the transmission unit 306 to shift to the pause state (pause period) (time t₁₂ to time t₁₆), and causes the receiving unit 308 to shift from the pause state to the receiving state of being capable of receiving a control signal from the receiving apparatus 7. Furthermore, the capsule controller 111 drives the antenna changeover switch unit 113 to electrically connect the receiving unit 308 and the transmitting and receiving antenna 112.

In a receivable period during which the capsule endoscope apparatus 11 can receive a control signal (time t₁₃ to time t₁₅), the receiving apparatus 7 causes the transmission unit 306 to transmit a control signal to the capsule endoscope apparatus 11 via any of the first antenna 41 to the eighth antenna 48 after the completion of receiving the wireless signal from the capsule endoscope apparatus 11. Furthermore, the receiving apparatus 7 transmits a control signal having the same content a plurality of times in the receivable period of the capsule endoscope apparatus 3 while switching sequentially among antennas to transmit a control signal via the antenna connection unit 6 at every specified timing. Furthermore, the capsule controller 111 causes the illumination unit 301 and the imaging unit 303 to light up and capture an image, respectively, (time t₁₄ to time t₁₆) in the pause period (time t₁₂ to time t₁₆) of the transmission unit 306.

According to the second embodiment of the present invention described above, the receiving unit 308 is activated to shift to the receiving state of being capable of receiving a control signal from the receiving apparatus 7 in the pause period from after the transmission unit 306 completes transmitting a wireless signal to starting transmitting the next wireless signal. As a consequence, it is not necessary to always supply power to the receiving unit 308. Accordingly, the power consumption of the capsule endoscope apparatus 11 can be reduced.

Furthermore, according to the second embodiment, the transmission unit 306 is up and running only during the transmission period when a wireless signal as image data is transmitted. Accordingly, it is possible to further reduce the power consumption of the capsule endoscope apparatus 11, and to reduce the capacity of the power supply unit 311 such as a battery to be mounted in the capsule endoscope apparatus 3. Accordingly, the size of the capsule endoscope apparatus 3 can be further reduced.

In the second embodiment, the illumination and imaging period and the wireless signal receivable period overlap with each other. However, the receivable period may be set shorter so that the receivable period does not overlap with the illumination and imaging period.

Moreover, in the second embodiment, each of the first antenna 41 to the eighth antenna 48 of the receiving apparatus 7 transmits and receives wireless signals and control signals. However, for example, a receiving antenna and a transmitting antenna may be provided separately.

### (Third Embodiment)

Next, a third embodiment of the present invention will be described. The third embodiment is different in the relationship between the transmission timing when the capsule endoscope apparatus according to the above-mentioned second embodiment transmits a wireless signal and the transmission timing when the receiving apparatus transmits a control signal. Hence, in the following, the relationship between the transmission timing when the capsule endoscope apparatus transmits a wireless signal and the transmission timing when the receiving apparatus transmits a control signal will be described. The same reference numerals will be used to refer to the same elements as those of the above-mentioned second embodiment.

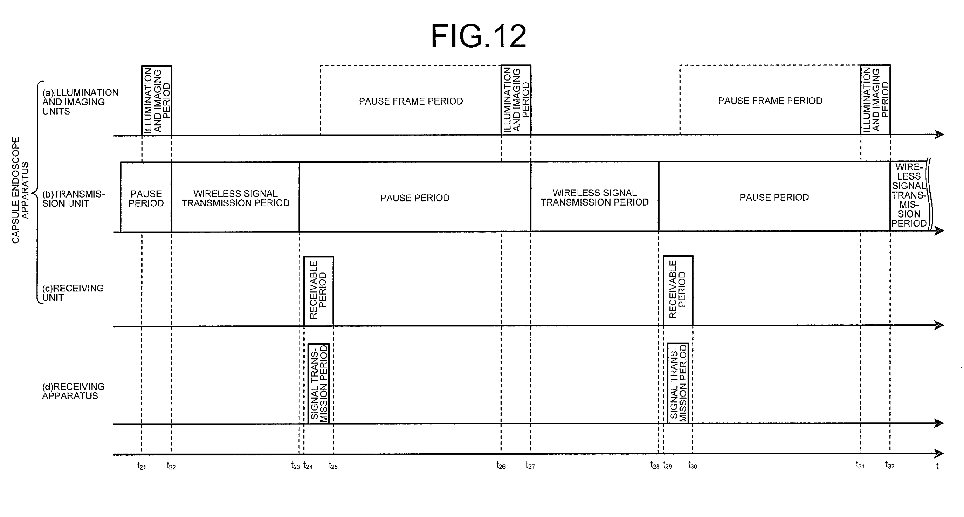

FIG. 12 is a timing chart illustrating the relationship between transmission timings when the capsule endoscope apparatus 11 transmits image data and transmission timings when the receiving apparatus 7 transmits a control signal. In FIG. 12, the horizontal axis indicates time. Moreover, FIG. 12(a) illustrates the operation timings of the imaging unit 303 and the illumination unit 301 of the capsule endoscope apparatus 11. FIG. 12(b) illustrates the transmission timings when the transmission unit 306 of the capsule endoscope apparatus 11 transmits image data. FIG. 12(c) illustrates the activation timings when the receiving unit 308 of the capsule endoscope apparatus 11 can receive a wireless signal. FIG. 12(d) illustrates the transmission timings when the receiving apparatus 7 transmits a control signal.

As illustrated in FIG. 12, if the capsule endoscope apparatus 11 can transmit image data captured by the imaging unit 303 at a specified frame rate (for example, up to four frames per second; 4 fps), the capsule controller 111 causes the transmission unit 306 to transmit the image data captured by the imaging unit 303 as a wireless signal via the transmitting and receiving antenna 112 (time t₂₂ to time t₂₃) after the completion of the operations in the illumination and imaging period (time t₂₁ to time t₂₂) when causing the imaging unit 303 to halt image capture in every other frame (for example, two frames per second; 2 fps).

Next, after the transmission unit 306 completes transmitting the wireless signal including the image data (time t₂₃), the capsule controller 111 causes the transmission unit 306 to shift to the pause state (time t₂₃ to time t₂₇), and causes the receiving unit 308 to shift from the pause state to the receiving state of being capable of receiving a control signal from the receiving apparatus 7 (time t₂₄ to time t₂₅). Furthermore, the capsule controller 111 drives the antenna changeover switch unit 113 to electrically connect the receiving unit 308 and the transmitting and receiving antenna 112.

In a period during which the capsule endoscope apparatus 11 can receive a control signal (time t₂₄ to time t₂₅), the receiving apparatus 7 causes the transmission unit 306 to transmit a control signal to the capsule endoscope apparatus 11 via any of the first antenna 41 to the eighth antenna 48 after the completion of receiving the wireless signal from the capsule endoscope apparatus 11. Furthermore, the receiving apparatus 7 transmits a control signal having the same content a plurality of times in the receivable period of the capsule endoscope apparatus 11 while sequentially switching antennas to transmit a control signal via the antenna connection unit 6 at every specified timing.

Next, at the end of the pause frame period (time t₂₆), the capsule endoscope apparatus 11 executes the illumination of the illumination unit 301 and the image capture of the imaging unit 303 (time t₂₆ to time t₂₇), and causes the transmission unit 306 to transmit a wireless signal including an image signal of one frame (time t₂₇ to time t₂₈).

According to the third embodiment of the present invention described above, the receiving unit 308 is activated to shift to the receiving state of being capable of receiving a control signal from the receiving apparatus 7 in the pause period during which the illumination unit 301 and the imaging unit 303 halts illumination and image capture in every other frame. As a consequence, it is not necessary to always supply power to the receiving unit 308. Accordingly, the power consumption of the capsule endoscope apparatus 11 can be reduced.

Furthermore, according to the third embodiment, it is possible to easily transition to the period during which the receiving unit 308 can receive a control signal from the receiving apparatus 7 in the pause frame period of the capsule endoscope apparatus 11.

In the third embodiment, the receiving state where the receiving unit 308 can receive a control signal from the receiving apparatus 7 may be continued during the pause frame period of the capsule endoscope apparatus 11.

### (Fourth Embodiment)

Next, a fourth embodiment of the present invention will be described. In the fourth embodiment, a guidance event setting process is performed via an image processing apparatus on a receiving apparatus having a function of notifying a subject of an event of an examination schedule (a guidance function). Hence, a description is given below of the event setting process of setting an event of the guidance of the receiving apparatus after the description of the configurations of the receiving apparatus and a cradle of the fourth embodiment. The same reference numerals will be used to refer to the same elements as those of the above-mentioned first embodiment.

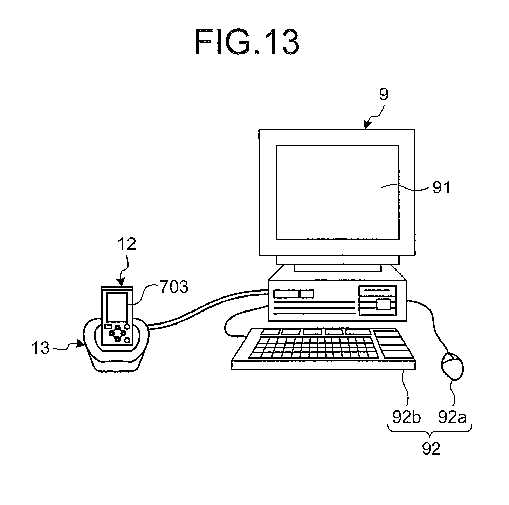

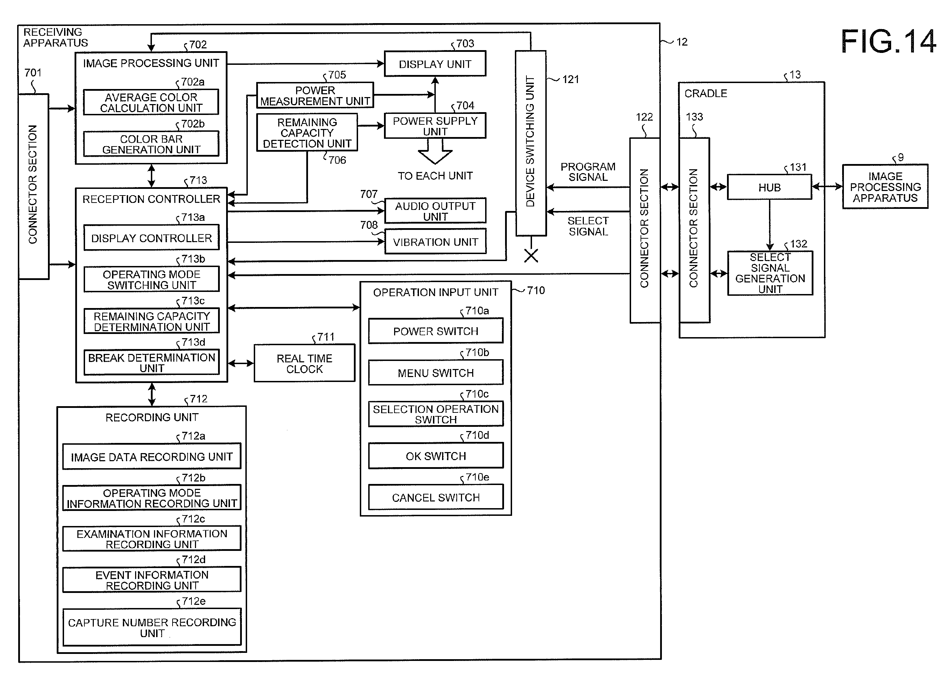

FIG. 13 is a schematic diagram illustrating general configurations of the receiving apparatus, the cradle, and the image processing apparatus according to the fourth embodiment. FIG. 14 is a block diagram illustrating functional configurations of the receiving apparatus and the cradle according to the fourth embodiment. The event setting process of setting an event of the guidance of the receiving apparatus is carried out before the start of an examination of the subject.

A receiving apparatus 12 illustrated in FIGS. 13 and 14 includes the connector section 701, the image processing unit 702, the display unit 703, the power supply unit 704, the power measurement unit 705, the remaining capacity detection unit 706, the audio output unit 707, the vibration unit 708, the operation input unit 710, the real time clock 711, the recording unit 712, the reception controller 713, a device switching unit 121, and a connector section 122.

The device switching unit 121 switches to a program device to be electrically connected to the image processing apparatus 9, for example, the reception controller 713 or the image processing unit 702, in response to an instruction signal input from the image processing apparatus 9 via a cradle 13.

The connector section 122 has a function as a communication interface, and performs transmission and reception bi-directionally to and from the image processing apparatus 9 via the cradle 13. Moreover, the connector section 122 is electrically connected to each of the device switching unit 121 and the reception controller 713.

Next, the cradle 13 will be described. The cradle 13 includes a hub 131, a select signal generation unit 132, and a connector section 133.

The hub 131 is configured using a USB connector, JTAG connector, LAN connector, or the like, and distributes a signal input from the image processing apparatus 9 to each of the connector section 133 and the select signal generation unit 132. Moreover, the hub 131 outputs, to the image processing apparatus 9, image data input via the receiving apparatus 12 and the connector section 133.

The select signal generation unit 132 generates a select signal to select a connection destination of the device switching unit 121 of the receiving apparatus 12 based on an instruction signal input via the hub 131, and outputs the select signal to the receiving apparatus 12 via the connector section 133.

The connector section 133 has a function as a communication interface, and outputs, to the receiving apparatus 12, the instruction signal distributed by the hub 131 and the select signal input from the select signal generation unit 132.

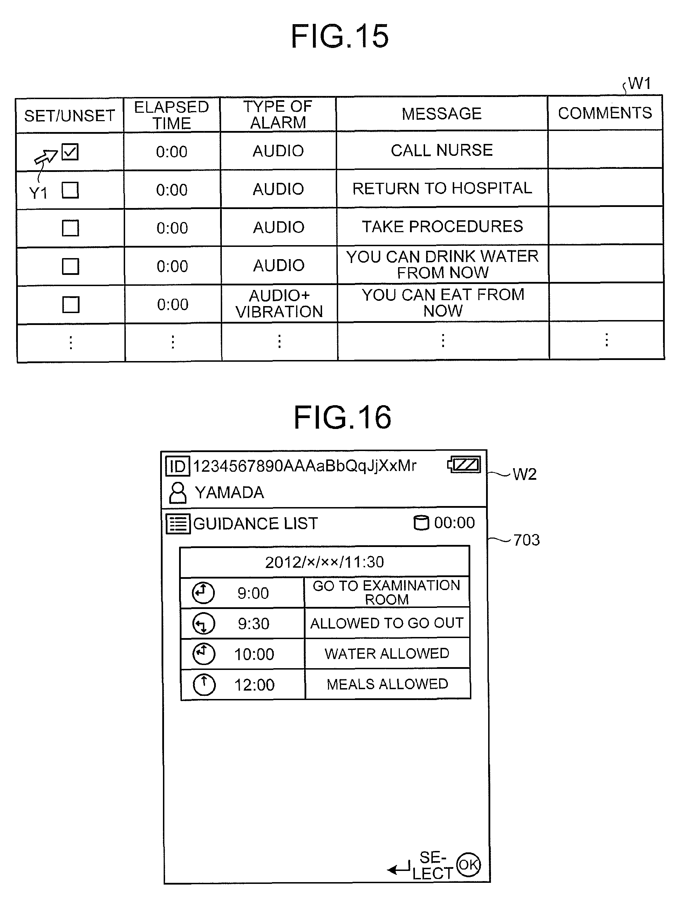

A description is given of the setting process of setting event information of the guidance on the receiving apparatus 12 having the above-mentioned configuration. FIG. 15 is a diagram illustrating an example of an event information setting screen for guidance by the receiving apparatus 12, the event information setting screen being displayed on the display unit 91 of the image processing apparatus 9.

Set/unset the guidance, elapsed time, the kind of alarm (sound and/or vibration), and a message and comments to be displayed are respectively displayed on an event information setting screen W1 illustrated in FIG. 15. A user uses the mouse 92a to move a cursor Y1 to a desired check box among check boxes corresponding to the guidance of the receiving apparatus 12, and clicks the mouse 92a to make a selection. Consequently, a check mark "v" is displayed in the check box. Furthermore, the user uses the mouse 92a to move the cursor Y1 to the elapsed time of the guidance to be set, clicks the mouse 92a to make a selection, and then uses the keyboard 92b to input elapsed time upon the occurrence of an event of the guidance. Furthermore, the user uses the mouse 92a and the keyboard 92b to set identification information of the subject 2 scheduled to undergo an examination and examination information, on the receiving apparatus 7.

In this manner, the guidance set in FIG. 15 is displayed on the display unit 703 of the receiving apparatus 12 as illustrated in FIG. 16. Specifically, a guidance list screen W2 illustrated in FIG. 16 contains the identification information of the subject 2 and a guidance list. For example, information to the effect that a message, "go to the examination room," is displayed on the display unit 703 at "9:00" is contained.

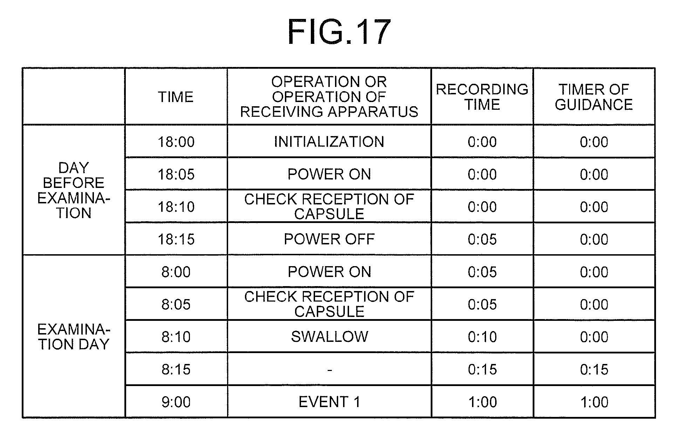

Next, a description is given of an initial setting process of the receiving apparatus 12 and the occurrence of a guidance event with reference to FIG. 17. FIG. 17 is a diagram illustrating an example of a time table of the initial setting process of the receiving apparatus 12 and the occurrence of the guidance event. In FIG. 17, a description is given of a case where the guidance of an event of the receiving apparatus 12 is given in one hour.

As illustrated in FIG. 17, the user carries out the initialization process on the receiving apparatus 12 on the day before the examination of the subject 2. Specifically, the user synchronizes the receiving apparatus 12 with the image processing apparatus 9 via the cradle 13 to synchronize the real time clock 711 of the receiving apparatus 12 with the time information of the image processing apparatus 9, and sets the above-mentioned event information on the receiving apparatus 12 using the image processing apparatus 9.

Next, the user turns on the power to the capsule endoscope apparatus 3 and the receiving apparatus 12, which are used for the examination, and checks the receiving apparatus 12's receiving state of a wireless signal including image data transmitted from the capsule endoscope apparatus 3. In this case, when having received a wireless signal from the capsule endoscope apparatus 3, the receiving apparatus 12 starts counting the recording time of an examination time, and checks reception for five minutes. At this point in time, the frame rate of the image data transmitted from the capsule endoscope apparatus 3 is preset (for example, 2 fps). Hence, the reception controller 713 starts counting the recording time of the examination time according to the number of images corresponding to the image data transmitted from the capsule endoscope apparatus 3.

After checking the receiving state, the user sets the power to the capsule endoscope apparatus 3 and the receiving apparatus 12 to an off state. In this case, the recording time of the receiving apparatus 12 is "00:05" (five minutes).

Next, on the day of the examination, the user turns on the power to the capsule endoscope apparatus 3 and the receiving apparatus 12, and checks the receiving apparatus 12's receiving state of a wireless signal including image data transmitted from the capsule endoscope apparatus 3. In this case, in terms of the recording time of image data, the receiving apparatus 12 adds the recording time starting from the recording time "00:05" regardless of the receiving state of the image data.

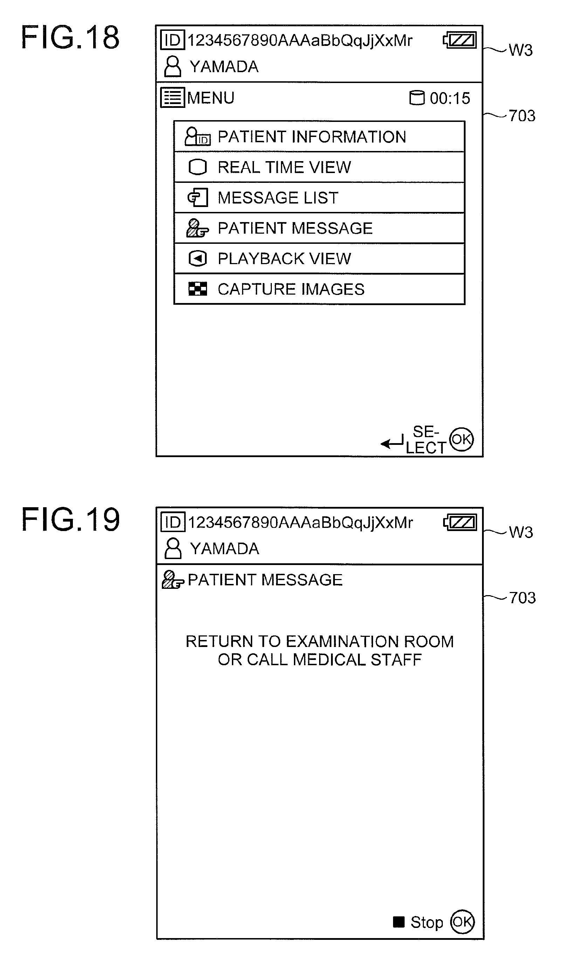

When the capsule endoscope apparatus 3 is subsequently swallowed by the subject 2 and a recording time "00:10" reaches a recording time "00:15," the receiving apparatus 12 sets the timer of the set guidance considering that 15 minutes have passed. At this point in time, a menu screen W3 illustrated in FIG. 18 is being displayed on the display unit 703 of the receiving apparatus 12. The identification information of the subject 2, for example, name and ID information, recording time, power state, patient information, and mode selection screen, are respectively displayed on the menu screen W3. Moreover, the user and medical staff can easily grasp an error in the event occurrence time since the recording time is displayed on the menu screen W3 in real time.

Next, when 45 minutes have passed since the start of the examination and the recording time of the receiving apparatus 12 has reached a recording time "01:00," the receiving apparatus 12 offers guidance for a set event 1. Specifically, as illustrated in FIG. 19, the receiving apparatus 12 displays "Return to the examination room or call medical staff" as a patient message on the display screen of the display unit 703.

According to the fourth embodiment described above, the recording time of the receiving apparatus 12 is counted before an examination based on the number of images, actual recording time is added to the recording time counted during the examination, the timer of an event is synchronized with the recording time after a lapse of a specified period of time, and the timer of the event is started. Consequently, it is possible to prevent a set event from occurring due to a reception check other than the one immediately before the examination.

Furthermore, according to the fourth embodiment, even if a check on the receiving state between the receiving apparatus 12 and the capsule endoscope apparatus 3 is interrupted, the examination of the subject 2 can be started without setting the event information again on the receiving apparatus 12.

Moreover, in the fourth embodiment, the image processing apparatus 9 may update a program of each of program devices, for example, an image processing unit 702 and the reception controller 713, of the receiving apparatus 12 via the cradle 13 as the initial setting process of the receiving apparatus 12. In this case, the image processing apparatus 9 outputs, to the cradle 13, a select signal to select a program device, for example, a select signal to select the reception controller 713, via the cradle 13. When having received the select signal from the image processing apparatus 9 via the hub 131, the select signal generation unit 132 of the cradle 13 generates a select signal to select the reception controller 713, outputs the select signal to the device switching unit 121, and connects the image processing apparatus 9 and the reception controller 713 in a manner of being capable of communicating bi-directionally. The image processing apparatus 9 subsequently transmits a program to update to the reception controller 713 via the cradle 13, and updates the program of the reception controller 713. Consequently, it is possible to keep a signal line used for a program of the connector section 122 of the receiving apparatus 12 to a minimum.

Furthermore, according to the fourth embodiment, the select signal generation unit 132 that generates a select signal to select a program device of the receiving apparatus 12 is provided in the cradle 13. Accordingly, the size of the receiving apparatus 12 can be reduced.

Furthermore, according to the fourth embodiment, the device switching unit 121 connects the image processing apparatus 9 to the program devices in the receiving apparatus 12 individually. Accordingly, it is possible to securely prevent a write of a wrong program to another program device.

Moreover, according to the fourth embodiment, the device switching unit 121 can prevent any program device from being connected to the image processing apparatus 9. Accordingly, it is possible to be realized as a program signal bus by known technologies such as USB and LAN.

### (First Modification of Fourth Embodiment)

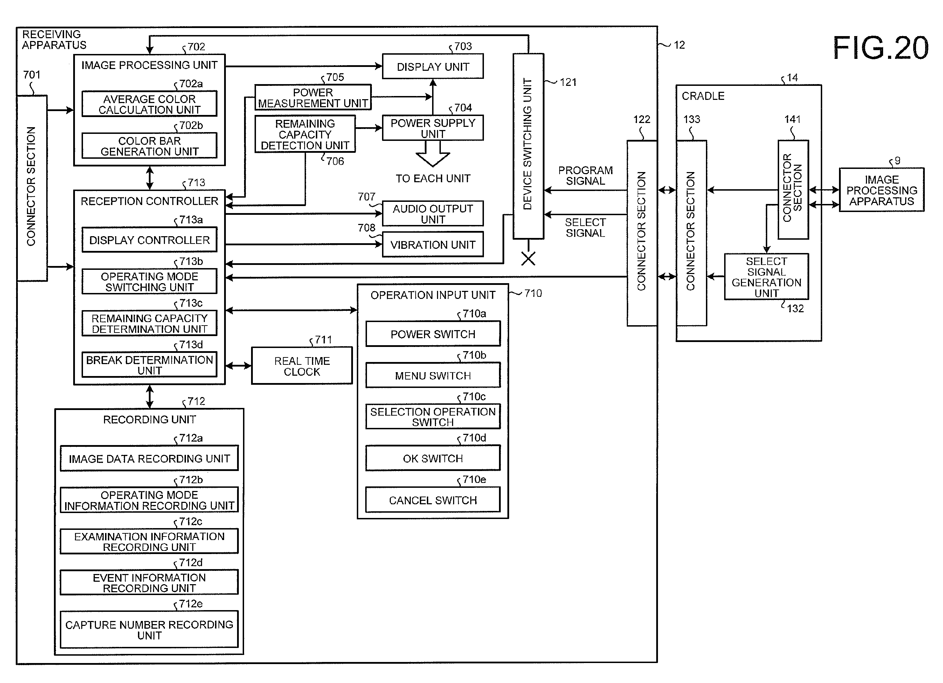

In the fourth embodiment, the configuration of the cradle can be changed. FIG. 20 is a block diagram illustrating functional configurations of a receiving apparatus and a cradle according to a first modification of the fourth embodiment.

As illustrated in FIG. 20, a cradle 14 includes the select signal generation unit 132, the connector section 133, and a connector section 141. The connector section 141 has a function as a communication interface, and outputs instruction signals and select signals input from the image processing apparatus 9, respectively, to the connector section 133 and the select signal generation unit 132.

According to the modification of the fourth embodiment described above, it is possible to keep a signal line used for a program of the connector section 122 of the receiving apparatus 12 to a minimum, and securely prevent a write of a wrong program to another program device.

### (Fifth Embodiment)

Next, a fifth embodiment of the present invention will be described. In the fifth embodiment, if an antenna cable that connects an antenna apparatus that receives a wireless signal transmitted from a capsule endoscope apparatus and an antenna connection unit is broken in the antenna apparatus and the antenna connection unit, the reception of a wireless signal transmitted from the capsule endoscope apparatus and the accuracy of detection of the position of the capsule endoscope apparatus inside a subject are deteriorated. Accordingly, the breaks of the antenna apparatus and the antenna connection unit are detected by a receiving apparatus. Furthermore, the antenna apparatus, the antenna connection unit, and the receiving apparatus according to the fifth embodiment have the same configurations as the antenna apparatus, the antenna connection unit, and the receiving apparatus according to the above-mentioned first embodiment, respectively. Hence, a description is given below of a process to be executed by the receiving apparatus when the antenna apparatus and the antenna connection unit are connected to the receiving apparatus. The same reference numerals will be used to refer to the same elements as those of the above-mentioned first embodiment.

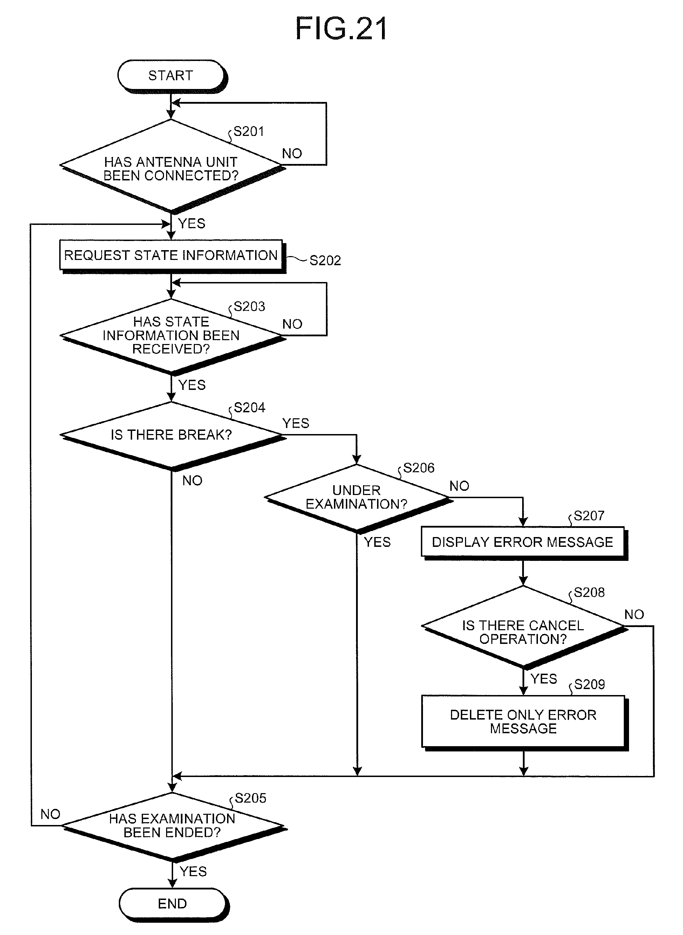

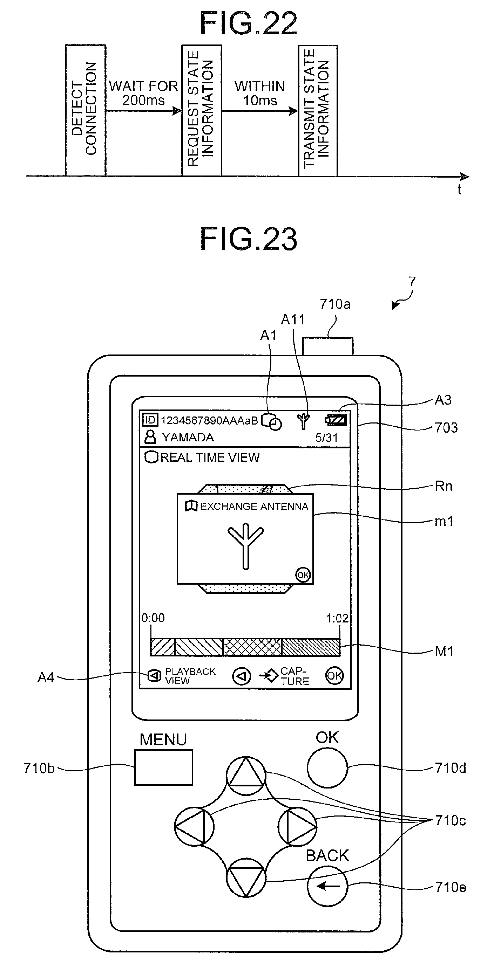

FIG. 21 is a flowchart illustrating an outline of a process to be executed by the receiving apparatus 7. FIG. 22 is a diagram illustrating timings when the receiving apparatus 7 acquires information from the antenna connection unit 6. In FIG. 22, the horizontal axis indicates time.

As illustrated in FIG. 21, the reception controller 713 judges whether or not the antenna connection unit 6 has been connected to the receiving apparatus 7 (Step S201). If the reception controller 713 judges that the antenna connection unit 6 has been connected to the receiving apparatus 7 (Step S201: Yes), the receiving apparatus 7 proceeds to Step S202. In contrast, if the reception controller 713 judges that the antenna connection unit 6 has not been connected to the receiving apparatus 7 (Step S201: No), the judgment is continued.

Next, the break determination unit 713d requests the state information of the antenna apparatus 4 from the antenna connection unit 6 (Step S202). Specifically, as illustrated in FIG. 22, the break determination unit 713d requests the state information of the antenna apparatus 4 and the antenna connection unit 6 from the antenna connection unit 6 after a lapse of 200 ms from the connection of the antenna connection unit 6 to the receiving apparatus 7. At this point in time, the antenna controller 609 transmits the state information of the antenna apparatus 4 to the receiving apparatus 7 within 10 ms.

If having subsequently received the state information (Step S203: Yes), the break determination unit 713d determines whether or not a break occurs in at least one or more of the first antenna 41 to the eighth antenna 48 of the antenna apparatus 4 (Step S204). Specifically, the break determination unit 713d determines whether or not information indicating that a break occurs in at least one or more of the first antenna 41 to the eighth antenna 48 has been added by the abnormal information addition unit 609b to the state information input from the antenna connection unit 6, and accordingly determines whether or not a break occurs in the antenna apparatus 4. If the break determination unit 713d determines that a break occurs in one or more of the first antenna 41 to the eighth antenna 48 of the antenna apparatus 4 (Step S204: Yes), the receiving apparatus 7 proceeds to Step S206 described below. In contrast, if the break determination unit 713d determines that a break occurs in none of the first antenna 41 to the eighth antenna 48 of the antenna apparatus 4 (Step S204: No), the receiving apparatus 7 proceeds to Step S205 described below.

In Step S205, the reception controller 713 judges whether or not the examination of the subject 2 has ended. Specifically, it is judged whether or not an instruction signal to end the examination has been input from the operation input unit 710. If the reception controller 713 judges that the examination of the subject 2 has ended (Step S205: Yes), the receiving apparatus 7 ends the process. In contrast, if the reception controller 713 judges that the examination of the subject 2 has not ended (Step S205: No), the receiving apparatus 7 returns to Step S202.

In Step S206, the reception controller 713 judges whether or not the subject 2 is under examination. Specifically, it is judged whether or not image data captured by the capsule endoscope apparatus 3 is being input from the antenna connection unit 6 via the antenna apparatus 4. If image data is being input, it is judged that the subject 2 is under examination. If the reception controller 713 judges that the subject 2 is under examination (Step S206: Yes), the receiving apparatus 7 proceeds to Step S205. In contrast, if the reception controller 713 judges that the subject 2 is not under examination (Step S206: No), the receiving apparatus 7 proceeds to Step S207.

Next, the display controller 713a causes the display unit 703 to display a warning (Step S207). Specifically, as illustrated in FIG. 23, the display controller 713a causes the display unit 703 to display an error message m1 and an error icon A11 indicating the break in the antenna apparatus 4. Consequently, the user can grasp the failure of the antenna apparatus 4 or the antenna connection unit 6.

Next, the reception controller 713 judges whether or not a cancel operation has been performed (Step S208). Specifically, the reception controller 713 judges whether or not an instruction signal to cancel the error message m1 has been input by the OK switch 710d being continuously pressed for a specified period of time (for example, three seconds). If the reception controller 713 judges that the cancel operation has been performed (Step S208: Yes), the receiving apparatus 7 proceeds to Step S209. In contrast, if the reception controller 713 judges that the cancel operation has not been performed (Step S208: No), the receiving apparatus 7 proceeds to Step S205.

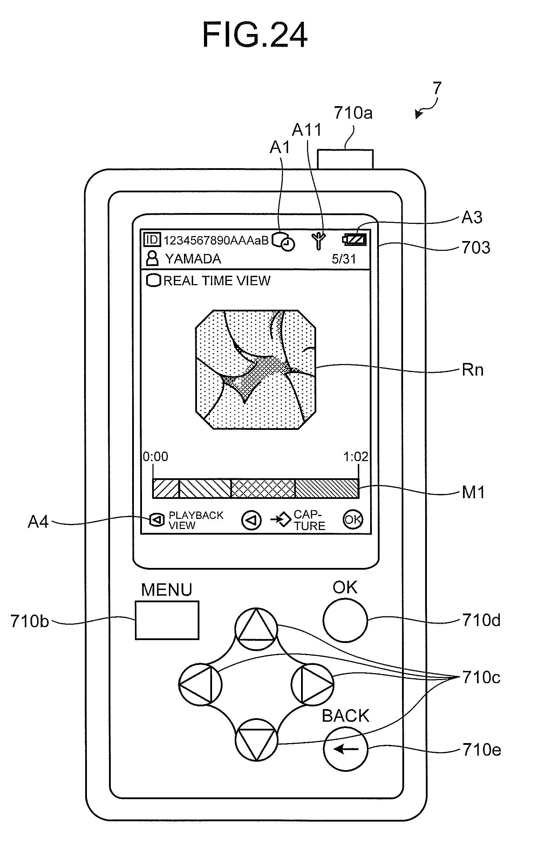

In Step S209, the display controller 713a deletes only the error message m1 from the display unit 703. At this point in time, as illustrated in FIG. 24, the display controller 713a deletes only the error message m1 and causes the display unit 703 to continue displaying the error icon A11. After Step S209, the receiving apparatus 7 proceeds to Step S205.

According to the fifth embodiment described above, an error message related to breaks in the antenna apparatus 4 and the antenna connection unit 6 is provided on the display unit 703 as a warning before the start of an examination. Accordingly, it is possible to prevent a useless examination from being carried out.

Furthermore, according to the fifth embodiment, if the OK switch 710d is continuously pressed for the specified period of time to input the instruction signal to delete the error message m1, the display controller 713a causes the display unit 703 to continue displaying only the error icon A11. Accordingly, it is possible to force the examination of the subject 2.

### (Sixth Embodiment)

Next, a sixth embodiment of the present invention will be described. In the sixth embodiment, the remaining capacity of a power supply unit of a receiving apparatus is determined to prevent image data from becoming unrecordable during an examination. If an image is displayed on a display unit for a long time, even necessary power to record image data transmitted by a capsule endoscope apparatus is consumed during the course of the examination. The receiving apparatus prevents a recording time to record image data from shortening due to the consumption. Furthermore, the receiving apparatus according to the sixth embodiment has the same configurations as the receiving apparatus according to the above-mentioned first embodiment. Hence, only a power determination process to be performed by the receiving apparatus will be described below. The same reference numerals will be used to refer to the same elements as those of the above-mentioned first embodiment.

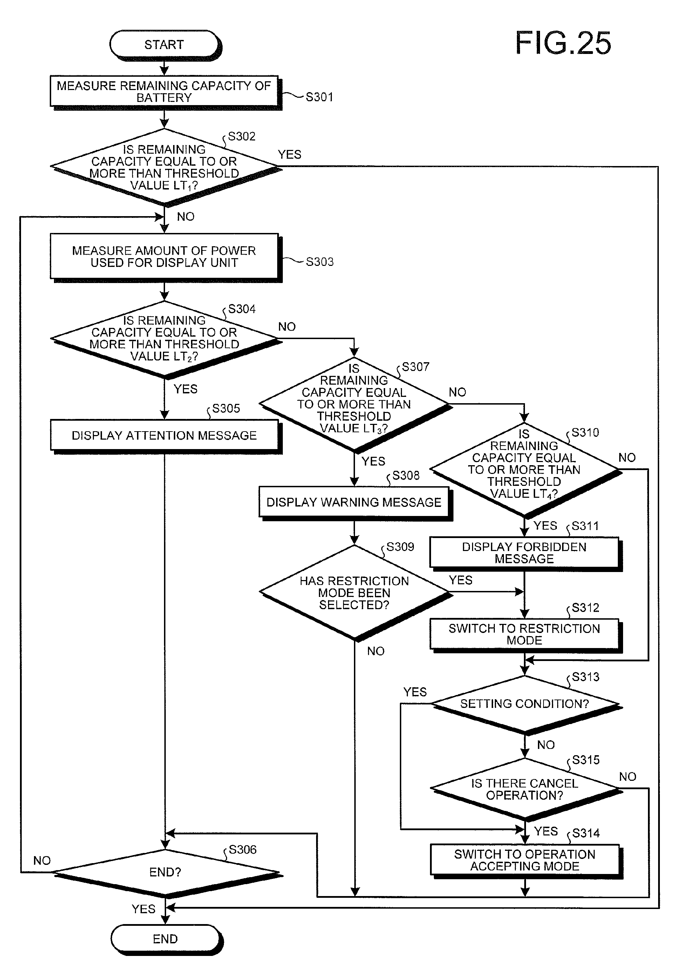

FIG. 25 is a flowchart illustrating an outline of the power determination process to be performed by the receiving apparatus 7. As illustrated in FIG. 25, the remaining capacity detection unit 706 measures the remaining power of the power supply unit 704 (Step S301). Specifically, the remaining capacity detection unit 706 measures the remaining capacity stored in the power supply unit 704 immediately after the receiving apparatus 7 is turned on.

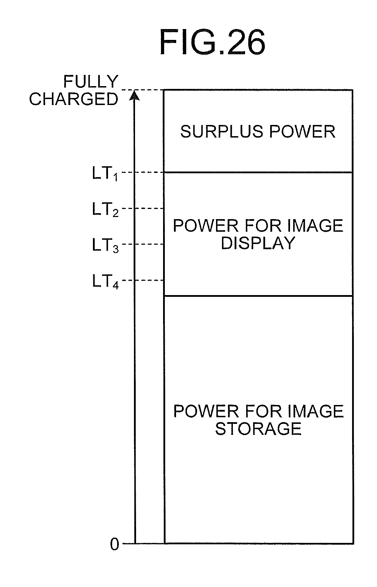

Next, the remaining capacity determination unit 713c determines whether or not the remaining amount of power of the power supply unit 704 detected by the remaining capacity detection unit 706 is equal to or more than a preset threshold value LT₁ (Step S302). Specifically, as illustrated in FIG. 26, the remaining capacity determination unit 713c determines whether or not, if the remaining capacity of the power supply unit 704 is divided into surplus power, power for image display, and power for image storage, the remaining capacity is equal to or more than the surplus (the threshold value TL₁). The power for image storage is power necessary to record image data in the course of the examination. Moreover, the power for image display is power necessary for estimated time for image display desired by the user. Furthermore, the surplus power is a surplus of power obtained by subtracting the power for image display and the power for image storage from power at the time of the power supply unit 704 is fully charged. The surplus power gradually reduces due to age deterioration of the power supply unit 704.

If the remaining capacity determination unit 713c determines that the remaining amount of power is equal to or more than the preset threshold value TL₁ (Step S302: Yes), the receiving apparatus 7 ends the process. In contrast, if the remaining capacity determination unit 713c determines that the remaining amount of power is not equal to or more than the preset threshold value TL₁ (less than the threshold value TL₁) (Step S302: No), the receiving apparatus 12 proceeds to Step S303.

Next, the power measurement unit 705 measures the amount of power supplied to the display unit 703 by the power supply unit 704 and accordingly measures the amount of power used by the display unit 703 (Step S303). Specifically, the display unit 703 is driven and accordingly the power measurement unit 705 measures the amount of power consumed by the display unit 703.

The remaining capacity determination unit 713c subsequently determines whether or not the remaining capacity of the power supply unit 704 obtained by subtracting the amount of power used that has been measured by the power measurement unit 705 from the amount of power of the power supply unit 704 measured by the remaining capacity detection unit 706 at the start of the examination is equal to or more than a threshold value TL₂ (Step S304). If the remaining capacity determination unit 713c determines that the remaining capacity of the power supply unit 704 is equal to or more than the threshold value TL₂ (Step S304: Yes), the receiving apparatus 7 proceeds to Step S305. In contrast, if the remaining capacity determination unit 713c determines that the remaining capacity of the power supply unit 704 is not equal to or more than the threshold value TL₂ (less than the threshold value TL₂) (Step S304: No), the receiving apparatus 7 proceeds to Step S307.

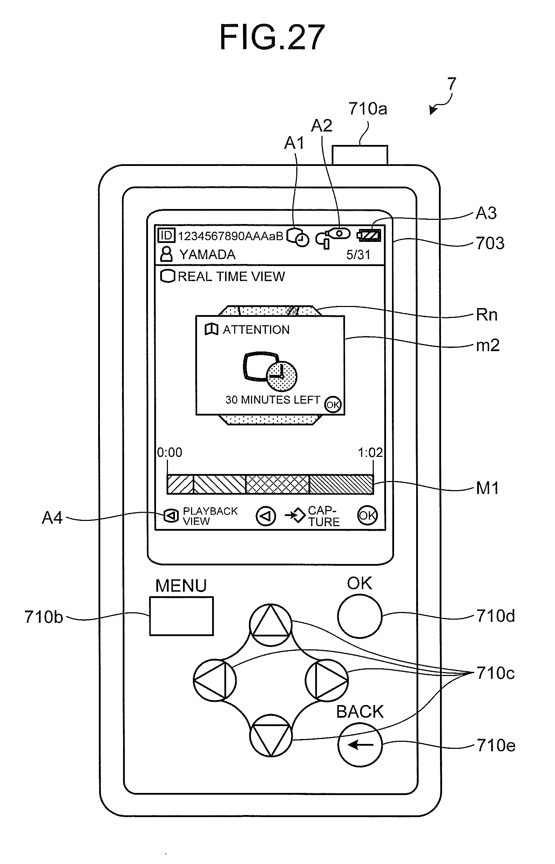

In Step S305, the display controller 713a causes the display unit 703 to display a message to pay attention to that a series of image data transmitted from the capsule endoscope apparatus 3 during the examination is to become unrecordable. Specifically, as illustrated in FIG. 27, the display controller 713a causes the display unit 703 to display an attention message m2.

Next, the reception controller 713 judges whether or not the examination has ended (Step S306). If the reception controller 713 judges that the examination has ended (Step S306: Yes), the receiving apparatus 7 ends the process. In contrast, if the reception controller 713 judges that the examination has not ended (Step S306: No), the receiving apparatus 7 returns to Step S303.

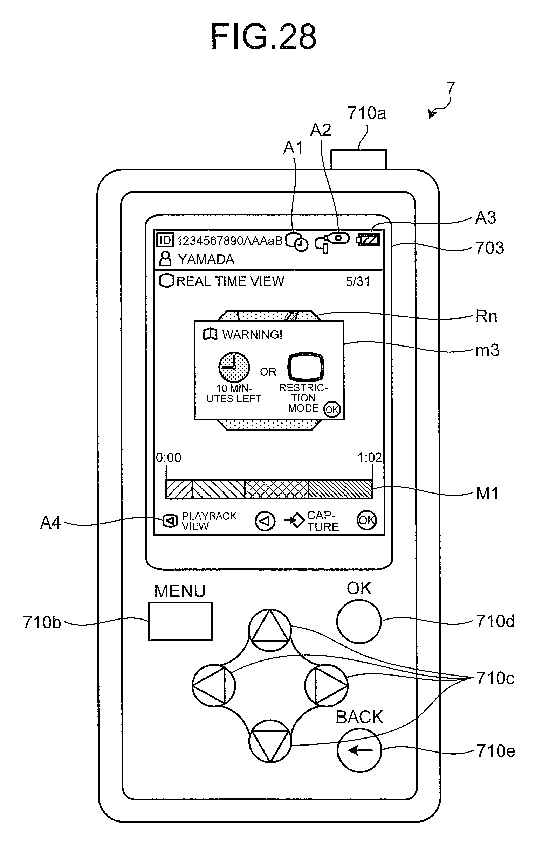

In Step S307, if the remaining capacity of the power supply unit 704 is equal to or more than a threshold value LT₃ (Step S307: Yes), the display controller 713a causes the display unit 703 to display a message to give a warning that a series of image data transmitted from the capsule endoscope apparatus 3 during the examination is to become unrecordanble (Step S308). Specifically, as illustrated in FIG. 28, the display controller 713a causes the display unit 703 to display a warning message m3 and a message to instruct a restriction mode.

Next, the reception controller 713 judges whether or not an instruction signal to switch the receiving apparatus 7 to the restriction mode has been input via the operation input unit 710 (Step S309). If the instruction signal to switch the receiving apparatus 7 to the restriction mode has been input (Step S309: Yes), the receiving apparatus 7 proceeds to Step S312 described below. In contrast, if the instruction signal to switch the receiving apparatus 7 to the restriction mode has not been input (Step S309: No), the receiving apparatus 7 proceeds to Step S306.

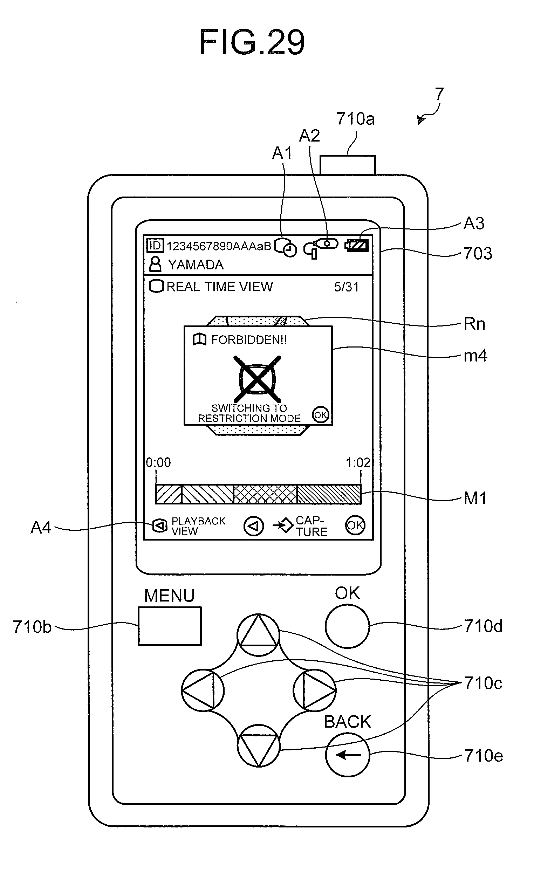

In Step S307, if the remaining capacity of the power supply unit 704 is not equal to or more than the threshold value LT₃ (less than the threshold value TL₃) (Step S307: No), and the remaining capacity of the power supply unit 704 is equal to or more than a threshold value LT₄ (Step S310: Yes), the display controller 713a causes the display unit 703 to display a message to forbid the acceptance of input at the receiving apparatus 7 (Step S311). Specifically, as illustrated in FIG. 29, the display controller 713a causes the display unit 703 to display a forbidden message m4 and information to the effect that the receiving apparatus 7 is switched to the restriction mode.

Next, the operating mode switching unit 713b switches from a mode to allow an arbitrary operation, the mode having been set for the receiving apparatus 7, to the restriction mode in which operation and display are restricted (Step S312). The restriction mode here includes a restriction to a frame rate at which the display unit 703 displays a real time view image (for example, 30 fps → 15 fps), a restriction to forbiddance of a real time view image displayed by the display unit 703 and the operation of the operation input unit 710 to accept input, a restriction to a capture image setting, and the like.

The reception controller 713 subsequently judges whether or not the receiving apparatus 7 has satisfied the setting condition (Step S313). The setting condition here indicate the number of recorded images corresponding to image data received from the capsule endoscope apparatus 3 by the receiving apparatus 7, elapsed time from the start of recording of image data by the receiving apparatus 7, or the remaining capacity of the power supply unit 704 of the receiving apparatus 7. For example, the reception controller 713 judges whether or not the number of recorded images corresponding to image data received from the capsule endoscope apparatus 3 by the receiving apparatus 7 has reached a specified number or more, or a specified period of time has passed since the image data received from the capsule endoscope apparatus 3 by the receiving apparatus 7 was recorded. If the reception controller 713 judges that the receiving apparatus 7 has satisfied the setting condition (Step S313: Yes), the receiving apparatus 7 proceeds to Step S314 described below. In contrast, if the reception controller 713 judges that the receiving apparatus 7 has not satisfied the setting condition (Step S313: No), the receiving apparatus 7 proceeds to Step S315 described below.

In Step S314, the operating mode switching unit 713b switches from the restriction mode set for the receiving apparatus 7 to the mode to allow an arbitrary operation. The receiving apparatus 7 proceeds to Step S306 after Step S313.

In Step S315, the reception controller 713 judges whether or not a cancel operation to cancel the restriction mode set for the receiving apparatus 7 has been performed. Specifically, the reception controller 713 judges whether or not the OK switch 710d of the operation input unit 710 has been pressed for a specified period of time (for example, three seconds) and accordingly judges whether or not an instruction signal to cancel the restriction mode set for the receiving apparatus 7 has been input. If the reception controller 713 judges that the cancel operation to cancel the restriction mode set for the receiving apparatus 7 has been performed (Step S315: Yes), the receiving apparatus 7 proceeds to Step S314. In contrast, if the reception controller 713 judges that the cancel operation to cancel the restriction mode set for the receiving apparatus 7 has not been performed (Step S315: No), the receiving apparatus 7 proceeds to Step S306.

In Step S307, if the remaining capacity of the power supply unit 704 is not equal to or more than the threshold value LT₃ (less than the threshold value TL₃) (Step S307: No), and the remaining capacity of the power supply unit 704 is not equal to or more than the threshold value LT₄ (less than the threshold value TL₄) (Step S310: No), the receiving apparatus 7 proceeds to Step S313.

According to the sixth embodiment described above, a series of image data during an examination can securely be recorded.

Furthermore, according to the sixth embodiment, a timing when measuring the remaining capacity of the power supply unit 704 is delayed to make it possible to extend the operating time of the display unit 703 and secure sufficient power for recording image data.

Furthermore, according to the sixth embodiment, a plurality of threshold values and a plurality of display contents are preset and the content of a message is changed in stages. Accordingly, the remaining capacity of the power supply unit 704 can be presented in detail to the user.

Moreover, according to the sixth embodiment, when the receiving apparatus 7 satisfies the setting condition, the mode of the receiving apparatus 7 is switched from the restriction mode to the operating mode to allow an arbitrary operation. Accordingly, an image can also be displayed on the display unit 703 at the end of the examination of the subject 2 as long as within the remaining capacity of the power supply unit 704, and therefore usability can be secured.

Moreover, according to the sixth embodiment, even if the restriction mode is set for the receiving apparatus 7, the restriction mode can be cancelled via the operation input unit 710. Accordingly, usability can be further secured.

In the sixth embodiment, the amount of power supplied to the display unit 703 is measured to determine the remaining capacity of the power supply unit 704. However, the remaining capacity of the power supply unit 704 may be determined, for example, by the driving time of the display unit 703. Furthermore, the driving time and driving power of each unit of the receiving apparatus 7 may be taken into consideration in accordance with the drive of the display unit 703 to determine the remaining capacity of the power supply unit 704.

Moreover, in the sixth embodiment, if the remaining capacity of the power supply unit 704 is equal to or more than the threshold value LT₄, the receiving apparatus 7 is switched to the restriction mode. However, the receiving apparatus 7 may be switched to the restriction mode, for example, at the threshold value LT₂ or more. Furthermore, the content of the restriction mode may be in accordance with each threshold value, for example, it may be switched to a restriction mode that reduces only the frame rate of an image displayed on the display unit 703 at the threshold value LT₂ or more.

### (Seventh Embodiment)

Next, a seventh embodiment of the present invention will be described. In the seventh embodiment, if a receiving apparatus is displaying in real time a real time view image corresponding to image data of the inside of a subject transmitted from a capsule endoscope apparatus, when an instruction signal to select a capture image is input from the OK switch 710d, the real time view image being displayed on the display unit 703 is set as the capture image. Moreover, the receiving apparatus according to the seventh embodiment has the same configurations as the above-mentioned first embodiment. Hence, a method in which the receiving apparatus sets a capture image will be described below. The same reference numerals will be used to refer to the same elements as those of the above-mentioned first embodiment.

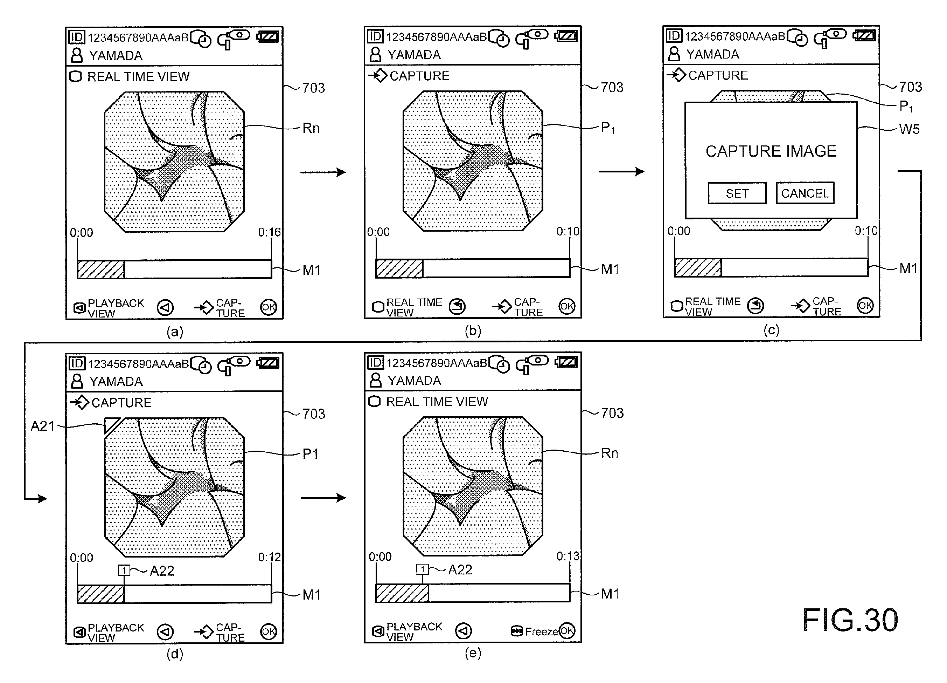

FIGS. 30 to 32 are diagrams illustrating examples of screen transition in the setting method for setting a capture image when the display unit 703 of the receiving apparatus 7 is displaying a real time view image.

As illustrated in FIG. 30(a), if the display unit 703 of the receiving apparatus 7 is displaying a real time view image Rₙ corresponding to image data of the inside of the subject 2 sequentially transmitted from the capsule endoscope apparatus 3, when an instruction signal to select a capture image is input from the OK switch 710d, the display controller 713a is switched to a capture image setting mode and causes the display unit 703 to display a current real time view image Rₙ as a capture image P₁ (FIG. 30(a) → FIG. 30(b)).

Next, the display controller 713a superimposes a capture image selection screen W5 on the capture image P₁ and causes the display unit 703 to display the capture image selection screen W5 (FIG. 30(b) → FIG. 30(c)).

If the display unit 703 is subsequently displaying the capture image selection screen W5 (FIG. 30(c)), when an instruction signal to set the capture image is input from the OK switch 710d, the reception controller 713 records a record number of the capture image P₁ being displayed on the display unit 703, the record number being recorded in the image data recording unit 712a, as a capture number in the capture number recording unit 712e. Specifically, the reception controller 713 associates a capture number of the capture image with a record number of the image data set as the capture image P₁, and records the capture number in the capture number recording unit. The record number here is a number indicating the order of recording, in the image data recording unit 712a, images corresponding to image data sequentially transmitted from the capsule endoscope apparatus 3 in the period of the course of the examination.

Next, the display controller 713a causes the display unit 703 to display, in the left area of the capture image P₁, a capture icon A21 indicating that the image data has been set as the capture image, and causes the display unit 703 to display, on the color bar M1, a capture number icon A22 at a position corresponding to the record number of the capture image (FIG. 30(d)).

After the capture icon A21 and the capture number icon A22 are displayed only for a specified period of time, the receiving apparatus 7 returns to a real time view mode that displays the real time view image Rₙ corresponding to the image data of the inside of the subject 2 sequentially transmitted from the capsule endoscope apparatus 3 (FIG. 30(e)).

In contrast, if the display unit 703 is displaying the capture image selection screen W5 (FIG. 30(c)), when an instruction signal to cancel the selected capture image P₁ is input from the cancel switch 710e, the receiving apparatus 7 returns to the real time view mode that displays the real time view image Rₙ corresponding to the image data of the inside of the subject 2 sequentially transmitted from the capsule endoscope apparatus 3 (FIG. 30(e)).

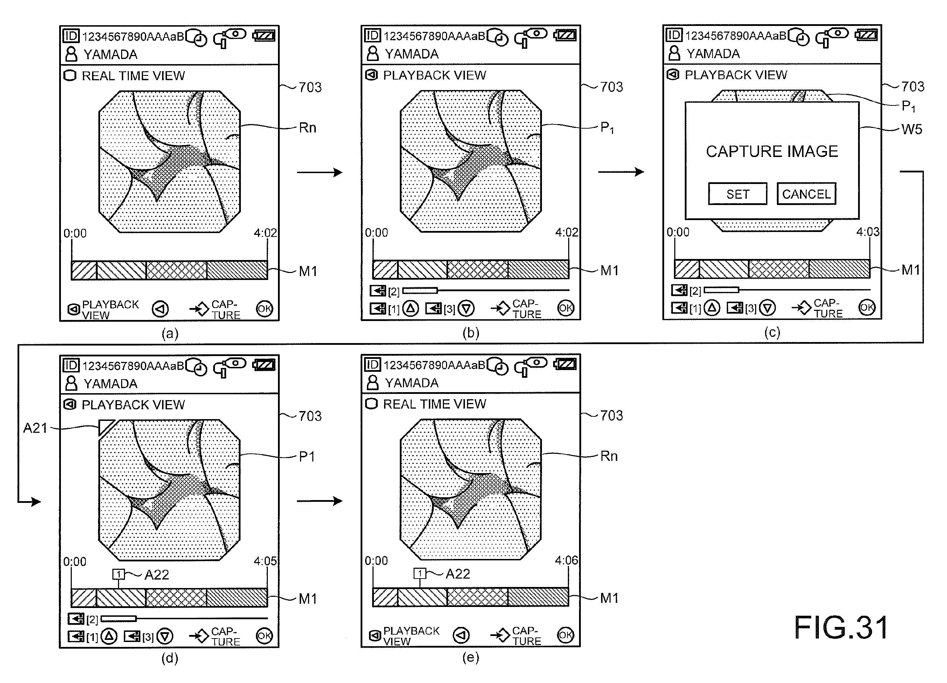

Moreover, as illustrated in FIG. 31(a), if the display unit 703 of the receiving apparatus 7 is displaying the real time view image Rₙ corresponding to the image data of the inside of the subject 2 sequentially transmitted from the capsule endoscope apparatus 3, when an instruction signal to switch to a playback view mode is input from the menu switch 710b, the display controller 713a causes the display unit 703 to display in playback view images corresponding to the image data recorded in the image data recording unit 712a (FIG. 31(a) → FIG. 31(b)).

Next, the display controller 713a superimposes the capture image selection screen W5 on the capture image P₁ and causes the display unit 703 to display the capture image selection screen W5 (FIG. 31(b) → FIG. 31(c)).

If the display unit 703 is displaying the capture image selection screen W5 in playback view mode (FIG. 31(c)), when an instruction signal to set the capture image P₁ is input from the OK switch 710d, the reception controller 713 records a record number of the capture image P₁ being displayed on the display unit 703, the record number being recorded in the image data recording unit 712a, as a capture number in the capture number recording unit 712e.

Next, the display controller 713a causes the display unit 703 to display, in the left area of the capture image P₁, the capture icon A21 indicating that the image data has been set as the capture image, and causes the display unit 703 to display, on the time bar M1, the capture number icon A22 at a position corresponding to the record number of the capture image (FIG. 31(d)).

After the capture icon A21 and the capture number icon A22 are displayed only for the specified period of time, the receiving apparatus 7 returns to the real time view mode that displays the real time view image Rₙ corresponding to the image data of the inside of the subject 2 sequentially transmitted from the capsule endoscope apparatus 3 (FIG. 31(e)).

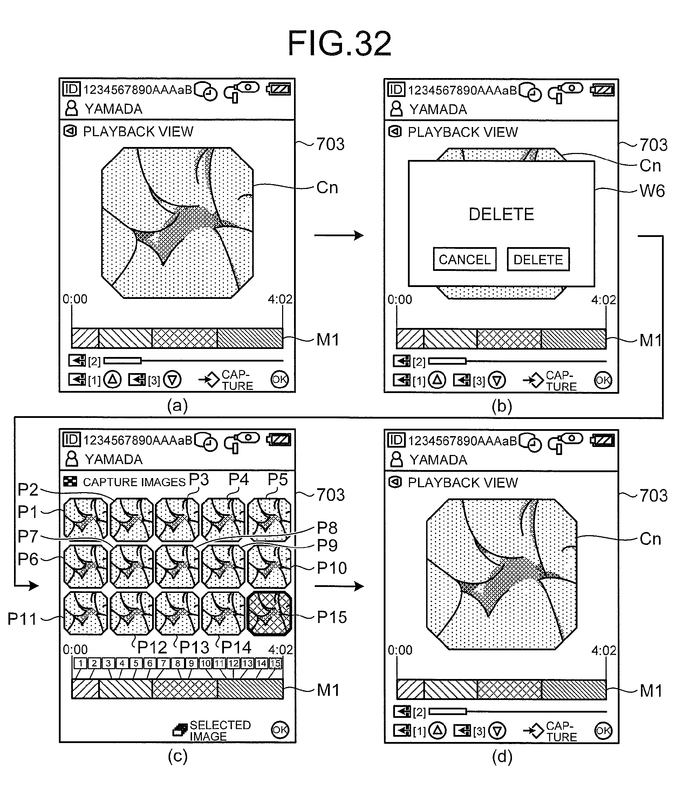

Moreover, as illustrated in FIG. 32(a), if the display unit 703 is displaying an image Cₙ (n = natural number) in playback view and an instruction signal to select a capture image is input from the OK switch 710d, when the recording capacity to record capture images is exceeded, the display controller 713a causes the display unit 703 to display a capture full screen W6 indicating that the recording capacity to record capture images is exceeded (FIG. 32(a) → FIG. 32(b)). At this point in time, if an instruction signal to stop selecting a capture image is input from the cancel switch 710e, the display controller 713a deletes the capture full screen W6. In contrast, if an instruction signal to set the capture image is input from the OK switch 710d, the display controller 713a causes the display unit 703 to display a list of set capture images P₁ to P₁₅ (FIG. 32(b) → FIG. 32(c)). At this point in time, the capture images P₁ to P₁₅ are displayed associated with their respective capture number icons on the color bar M1.

The display controller 713a subsequently highlights the selected capture image P₁₅ in response to the instruction signal input from the selection operation switch 710c. At this point in time, if an instruction signal to delete the capture image is input from the OK switch 710d, a record number of the selected capture image P₁₅ is deleted from the capture number recording unit to return to the image Cₙ on the playback view screen (FIG. 32(c) → FIG. 32(d)).

In this manner, a capture image is set for a plurality of pieces of image data recorded in the receiving apparatus 7 in the course of the examination. Accordingly, if the receiving apparatus 7 is connected to the image processing apparatus 9 via the cradle 8 to download the series of image data, when the image processing apparatus 9 displays the series of image data, the capture image is displayed as a thumbnail image.

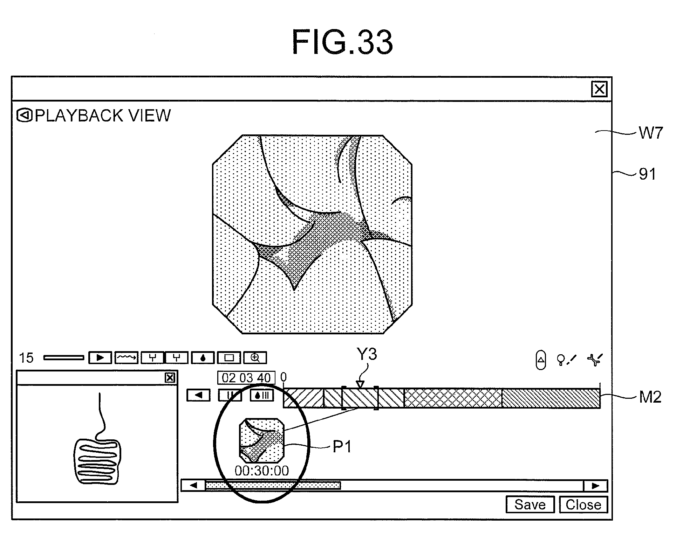

FIG. 33 is a diagram illustrating an example of an examination screen of the subject 2 displayed by the image processing apparatus 9. As illustrated in FIG. 33, the image processing apparatus 9 displays an examination screen W7. At least an image corresponding to image data captured in the subject 2, a color bar M2 created based on brightness information included in the series of image data, the capture image P₁ (the thumbnail image) displayed while associated with the color bar M2, icons to accept the input of instruction signals to instruct various operations, and a subject image for estimating the position of the capsule endoscope apparatus 3 are respectively displayed on the examination screen W7. The user uses the mouse 92a or the keyboard 92b to check images before and after the capture image, and set the first image of the small intestine among the series of image data. Accordingly, it is possible to bring efficiency to the observation of the subject 2.

According to the seventh embodiment described above, the receiving apparatus 7 associates a record number corresponding to a capture image with a series of image data, and records the record number. Accordingly, when the user observes the subject 2 on the image processing apparatus 9, it is possible to easily add a landmark to an image on the boundary between organs of the subject 2, and to bring efficiency to the observation of the subject 2.

Moreover, in the seventh embodiment, if the receiving apparatus 7 sets a capture image or checks an image in playback view mode, going back through the series of image data sequentially transmitted from the capsule endoscope apparatus 3 and recorded, it is also possible to change a display interval to display, on the display unit 703, the series of image data recorded in the image data recording unit 712a. Furthermore, the receiving apparatus 7 can change the display area and display density of the color bar M1 in synchronization with the display interval.

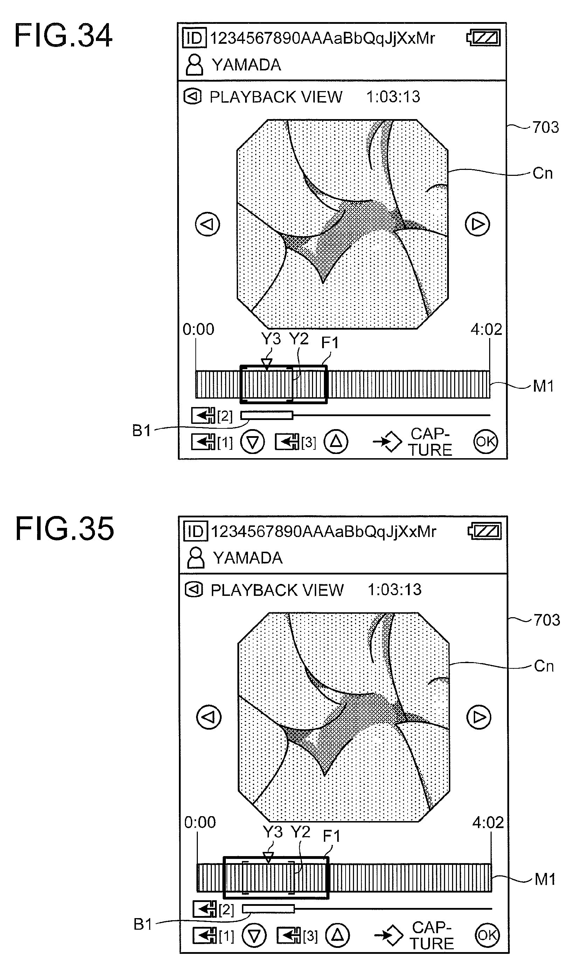

FIG. 34 is a diagram illustrating an example of a playback view screen displayed in playback view mode by the display unit 703 of the receiving apparatus 7.

As illustrated in FIG. 34, at least the image Cₙ captured in the subject 2, the color bar M1, various icons, a data range frame F1 indicating a currently selected data range, a selection cursor Y2 to select the area of images to display in the data range frame F1, an indicator Y3 indicating a current display position on the color bar M1, and a scroll bar B1 to scroll within the data range in synchronization with the selection cursor Y2 are respectively displayed on the playback view screen. In this case, if a right or left button of the selection operation switch 710c is operated, the display controller 713a moves an image after 30 minutes and the position of the scroll bar to cause the display unit 703 to carry out display (see FIG. 35).

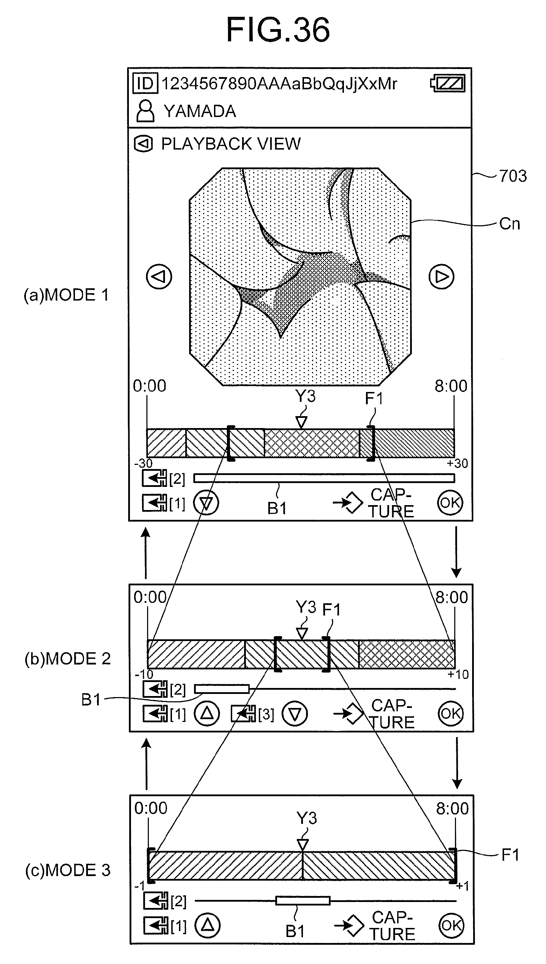

Moreover, as illustrated in FIG. 36, if the display unit 703 is displaying the image Cₙ in playback view mode (FIG. 36(a)), when a down or up button of the selection operation switch 710c is operated, the receiving apparatus 7 switches modes to change the display interval to display image data, the display area of the color bar and the display density of each section of the bar in the order of mode 1, mode 2, and mode 3 and causes the display unit 703 to carry out display (FIG. 36(a) → FIG. 36(b) → FIG. 36(c)). At this point in time, the display controller 713a changes the display interval, and the display area and display density of the color bar of an average color according to the mode, and causes the display unit 703 to carry out display (for example, ±30 minutes → ±10 minutes → ±5 minutes).

In this manner, image display can be performed according to the examination time. Accordingly, a desired image can easily be retrieved regardless of the examination time.

(Other Embodiments)

Moreover, in the present invention, a plurality of antennas is arranged on one sheet-shaped plate, but may be arranged individually, for example, at specified positions on the body surface of a subject.

Moreover, in the present invention, the description has been given taking an open-end antenna as an example. However, the kind of antenna is not particularly limited, and may be, for example, a loop antenna. Furthermore, an active circuit that amplifies a wireless signal may be provided to each antenna as appropriate.

Further effects and modifications can easily be derived by those skilled in the art. Hence, a wider aspect of the present invention is not limited to the specific details and the representative embodiments, which are expressed and described above. Therefore, various modifications can be made without departing from the spirit or scope of the overall concept of the invention defined by the accompanying claims and their equivalents.

### Reference Signs List

- 1: Capsule endoscope system
- 2: Subject
- 3, 11: Capsule endoscope apparatus
- 4: Antenna apparatus
- 5: Antenna cable
- 6: Antenna connection unit
- 7, 12: Receiving apparatus
- 8, 13, 14: Cradle
- 9: Image processing apparatus
- 91, 703: Display unit
- 92: Operation input device
- 92b: Keyboard
- 92a: Mouse
- 111, 312: Capsule controller
- 111a, 312a: Antenna switching unit
- 112: Transmitting and Receiving antenna
- 113, 601: Antenna changeover selection switch unit
- 121: Device switching unit
- 122, 133, 141, 600, 608, 701, 709: Connector section
- 131: Hub
- 132: Select signal generation unit
- 301: Illumination unit
- 302: Illumination drive unit
- 303: Imaging unit
- 304: Imaging drive unit
- 305, 603: Signal processing unit
- 306, 605: Transmission unit
- 307: Transmitting antenna
- 308, 602: Receiving unit
- 309: Receiving antenna
- 310, 607, 712: Recording unit
- 311, 704: Power supply unit
- 312b: Signal determination unit
- 312c: Operating mode switching unit
- 604: Received electric field strength detection unit
- 606: Break detection unit
- 609: Antenna controller
- 609a: Selection controller
- 609b: Abnormal information addition unit
- 702: Image processing unit
- 702a: Average color calculation unit
- 702b: Color bar generation unit
- 705: Power measurement unit
- 706: Remaining capacity detection unit
- 707: Audio output unit
- 708: Vibration unit
- 710: Operation input unit
- 711: Real time clock
- 713: Reception controller
- 713a: Display controller
- 713b: Operating mode switching unit
- 713c: Remaining capacity determination unit
- 713d: Break determination unit

## Claims

1. A capsule endoscope apparatus that is configured to be introduced into a subject to acquire information on an inside of the subject and to wirelessly communicate with a receiving apparatus provided outside the capsule endoscope apparatus, the capsule endoscope apparatus comprising:
an imaging unit configured to capture images of the subject and generate image data of the inside of the subject;
a transmission unit configured to transmit a wireless signal including the image data, outside the capsule endoscope apparatus;
a receiving unit configured to receive a control signal for instructing an operation of the capsule endoscope apparatus, the control signal having been transmitted from the receiving apparatus; and
a capsule controller configured to activate the receiving unit in synchronization with a transmission timing when the transmission unit transmits the wireless signal.

2. The capsule endoscope apparatus according to claim 1, wherein the capsule controller activates the receiving unit immediately after the transmission unit starts transmitting the wireless signal.

3. The capsule endoscope apparatus according to claim 1, wherein the capsule controller activates the receiving unit after the transmission unit completes transmission of the wireless signal.

4. The capsule endoscope apparatus according to claim 1, wherein
the imaging unit is configured to capture the images at a specified frame rate, and
the capsule controller is configured to decide the number of times of activation to activate the receiving unit in accordance with the specified frame rate.

5. The capsule endoscope apparatus according to claim 1, wherein the capsule controller is configured to cause the imaging unit to halt image capture at specified frame intervals, and activate the receiving unit in a pause period during which the imaging unit pauses.

6. The capsule endoscope apparatus according to claim 1, wherein when the receiving unit receives the control signal having the same instruction content a plurality of times, the capsule controller causes the capsule endoscope apparatus to execute an operation in accordance with the control signal only once.

7. A capsule endoscope system comprising:
a capsule endoscope apparatus configured to be introduced into a subject to acquire information on an inside of the subject; and
a receiving apparatus configured to wirelessly communicate with the capsule endoscope apparatus, wherein
the capsule endoscope apparatus includes:
an imaging unit configured to capture images of the subject and generate image data of the inside of the subject;
a transmission unit configured to transmit a wireless signal including the image data, outside the capsule endoscope apparatus;
a receiving unit configured to receive a control signal from the receiving apparatus; and
a capsule controller configured to activate the receiving unit in synchronization with a transmission timing when the transmission unit transmits the wireless signal, and
the receiving apparatus includes:
a receiving unit configured to receive the wireless signal;
a transmission unit configured to transmit the control signal; and
a reception controller configured to cause the transmission unit to transmit the control signal having the same instruction content a plurality of times at specified intervals immediately after the receiving unit receives the wireless signal.

8. A receiving apparatus to which an antenna apparatus having a plurality of antennas is detachably attached, the receiving apparatus comprising:
an antenna selector configured to select one antenna from among the plurality of antennas for receiving a wireless signal transmitted from outside when the antenna apparatus is attached; and
a break determination unit configured to determine whether or not a break occurs in the antenna selected by the antenna selector.

9. The receiving apparatus according to claim 8, comprising:
a display unit configured to display an image; and
a display controller configured to cause the display unit to display break information indicating that the break occurs in the selected antenna if the break determination unit determines that the break occurs in the selected antenna.

10. The receiving apparatus according to claim 9, further comprising an operation input unit configured to receive input of an instruction signal to delete the break information, wherein
the display controller causes the display unit to delete only the break information when the instruction signal is input.

11. A receiving apparatus for receiving a wireless signal having information on an inside of a subject transmitted from a capsule endoscope apparatus introduced into the subject, the receiving apparatus comprising:
a power supply unit configured to supply power to component parts of the receiving apparatus;
a display unit configured to display an image corresponding to image data;
a remaining capacity detection unit configured to detect remaining capacity stored in the power supply unit;
a remaining capacity determination unit configured to determine whether or not the remaining capacity detected by the remaining capacity detection unit is equal to or more than a preset threshold value; and
a display controller configured to cause the display unit to display a warning when the remaining capacity determination unit determines that the remaining capacity is less than the threshold value.

12. The receiving apparatus according to claim 11, further comprising a power measurement unit configured to measure power consumption consumed by the display unit, wherein
the remaining capacity determination unit is configured to determine whether or not remaining capacity obtained by subtracting the power consumption measured by the power measurement unit from the remaining capacity detected by the remaining capacity detection unit at a start of an examination is equal to or more than the threshold value.

13. The receiving apparatus according to claim 12, wherein the power measurement unit starts measuring the power consumption when the remaining capacity determination unit determines that the remaining capacity is less than the threshold value.

14. The receiving apparatus according to claim 13, further comprising an operating mode switching unit configured to switch to one of an arbitrary operating mode to allow an arbitrary operation and a restriction mode in which operation is restricted, to set for the receiving apparatus, wherein
the operating mode switching unit sets the receiving apparatus in the restriction mode when the remaining capacity determination unit determines that the remaining capacity of the power supply unit is less than the threshold value.

15. The receiving apparatus according to claim 14, further comprising an operation input unit configured to receive input of an instruction signal to set a setting condition for the receiving apparatus before the start of the examination, wherein
the operating mode switching unit switches from the restriction mode to the arbitrary operating mode when the receiving apparatus satisfies the setting condition.

16. The receiving apparatus according to claim 15, further comprising a recording unit configured to record the image data, wherein
the setting condition is any of data amount of the image data to be recorded by the recording unit, the remaining capacity of the power supply unit, and elapsed time from execution of the examination.
